# EUROPEAN PATENT APPLICATION

(11) **EP 1 101 820 A1**
(43) Date of publication of application: **23.05.2001**
(21) Application number: 99122666.3
(22) Date of filing: 15.11.1999
(51) Int. Cl.: C12N 15/18, C07K 14/475, A61K 38/18, C07K 16/22, C07K 16/28

(54) **Pentraxin I and Pentraxin receptor, inhibitors of said proteins and pharmaceutical compositions containing said compounds**

(71) Applicant: MERCK PATENT GmbH, 64293 Darmstadt (DE)
(72) Inventor: Messeguer Peypoch Ramon, 08330-Premià de Mar (Barcelona) (ES); Rosell Vives Elisabet, 08008-Barcelona (ES); Martinez Escolà Josep M, 08017-Barcelona (ES); Rodés Gubern Blanca, 08849-S. Climent de Llobregat, Barcelona (ES); Adàn Plana Jaume, 08301-Mataro ( Barcelona ) (ES); Puig Calvo Nuria, 08027-Barcelona (ES); Carceller Rosa Ana, 08028- Barcelona (ES); Masa Alvarez Marc, 08020-Barcelona (ES); Piulats Xanco', 08030-Barcelona (ES); Den Daas Izaak, 64407 Fränkisch-Crumbach (DE); Trullàs Oliva Ramon, 08226-Terrassa (ES); DeGregorio-Rocasolano Barbany Nuria, 08921- Santa Coloma de Gramanet (ES)
(74) Representative: Schüssler, Andrea, Dr.

(57) **Abstract**

Described are nucleic acid sequences encoding the human Pentraxin receptor and related proteins as well as a pharmaceutical composition comprising a therapeutically effective amount of (a) Pentraxin I or the human Pentraxin receptor, (b) a nonfunctional variant of the protein of (a), (c) an antibody to the protein of (a) or (b), (d) a nucleic acid sequence encoding the protein of (a) or (b), (e) an antisense RNA sequence characterized in that it is complementary to an Pentraxin I or human Pentraxin receptor mRNA and can selectively bind to said mRNA, said sequence being capable of inhibiting the synthesis of Pentraxin I or the human Pentraxin receptor, or (f) a ribozyme characterized in that it is complementary to an Pentraxin I or human Pentraxin receptor mRNA and can selectively bind to and cleave said mRNA, thus inhibiting the synthesis of Pentraxin I or the human Pentraxin receptor. Moreover, the use of the above compounds for treating neuronal disorders or providing a neuroprotective effect is described. The above compounds are also useful for detecting a disease associated with a neuronal disorder based on a deregulated expression of the above proteins or the presence of Pentraxin I/human Pentraxin receptor having no or at least reduced biological activity.

### Annex to the application documents - subsequently filed sequences listing

SEQUENCE LISTING

<110> Merck Patent GmbH

<120> Pentraxin I and Pentraxin receptor, inhibitors of said
      proteins and pharmaceutical compositions containing
      said compounds

<130> M 4331

<140> EP 99122666.3
<141> 1999-11-15

<160> 2

<170> PatentIn Ver. 2.1

<210> 1
<211> 1680
<212> DNA
<213> Homo sapiens

<400> 1
   ggcctgatag cgcggcgtgt ggaccggccg aagagcgcgc ccagagcggc gccgtcgcga 60
   gccacagccc gagccggtcc cagccgagcc gagccccagc cgagccgagc cggcccgagc 120
   gctccggtgc ccgcagccat gccggccggc cgcgcgcgca cctgtgcgct gctcgccctc 180
   tgcctcctgg ggccccagga tttcgggccg acgcgcttca tctgcacttc ggtgccggtg 240
   gacgccgaca tgtgtgccgc gtccgtggcc gcgggcggcg cggaggagct ccggagcagc 300
   aatgtgctgc agctccggga gaccgtgctg cagcagaagg agaccatcct gagccagaag 360
   gagaccatcc gcgagctgac cgccaagctg ggccgctgcg agagccagag cacgctggac 420
   cccggagccg gcgaggcccg ggcgggcggc ggccgcaagc agccgggctc gggcaagaac 480
   accatgggcg acctgtcccg gacaccggcc gccgagacgc tcagccaact cgggcaaact 540
   ttgcaatcgc tcaaaacccg cctggagaac ctcgagcagt acagccgcct caattcctcc 600
   agccagacca acagcctcaa ggatctgctg cagagcaaga tcgatgagct ggagaggcag 660
   gtgctgtccc gggtgaacac cctggaggag ggcaaggggg ggcccaagaa cgacaccgag 720
   gagagggtca agatcgagac cgccctgacc tccctgcacc agcggatcag cgagctcgag 780
   aaaggtcaga aagacaaccg ccctggagac aagttccagc tcacattccc actgcggacc 840
   aactatatgt atgccaaggt gaagaagagc ctgccagaga tgtacgcctt cactgtctgc 900
   atgtggctca agtccagcgc cacgccaggt gtgggcacgc ccttctccta cgctgtgccc 960
   ggccaggcca acgagctggt cctcattgag tggggcaaca accccatgga gatcctcatc 1020
   aatgacaagg tggccaagct gccttttgtc atcaatgatg gcaagtggca ccacatctgt 1080
   gtcacctgga ccacccggga cggggtctgg gaggcctacc aggatggcac gcagggtggc 1140
   agtggcgaga acttggcgcc ctatcacccc atcaagcccc agggcgtgct ggtgctgggc 1200
   caggagcagg acactctggg tggtgggttt gatgccaccc aggcatttgt gggtgagctg 1260
   gcccacttca acatctggga ccgcaagctg acccccgggg aggtgtacaa cctggccacc 1320
   tgcagcacca aggctctgtc cggcaatgtc atcgcctggg ctgaatccca catcgagatc 1380
   tacggcgggg ccaccaagtg gaccttcgag gcctgtcgcc agatcaactg agcacggcag 1440
   gccaggctga gccgcccgcc ctcgccccct gcttgtgcgg cgatgatctg tttgtgcgtc 1500
   tcttctctcc cttttcccca ggaatgaacc gaggccgtcg ccctgcacac gcacacgcac 1560
   acagcctggt tttgtcctca tgcacacgaa gcagcccctg ctcccatctg tccctgagga 1620
   agccccactt ctctgtagga gcccggactc tctcagcatg ccccattcac agctgaagtg 1680

<210> 2
<211> 1853
<212> DNA
<213> Homo sapiens

<400> 2

agagcggcgg cagcagcggc cgcggcgaca gctccagctc cggctccggc tccggctccg 60
gctccggctc ccgcgcctgc cccgctcggc ccagcgcgcc cgggctccgc gccccgaccc 120
cgccgccgcg cctgccgggg gcctcgggcg cccccgccgc ccgcctcacg ctgaagttcc 180
tggccgtgct gctggccgcg ggcatgctgg cgttcctcgg tgccgtcatc tgcatcatcg 240
ccagcgtgcc cctggcggcc agcccggcgc gggcgctgcc cggcggcgcc gacaatgctt 300
cggtcgcctc gggcgccgcc gcgtccccgg gcccgcagcg gagcctgagc gcgctgcacg 360
gcgcgggcgg ttcagccggg ccccccgcgc tgcccggggc acccgcggcc agcgcgcacc 420
cgctgccgcc cgggcccctg ttcagccgct tcctgtgcac gccgctggct gctgcctgcc 480
cgtcgggggc ccagcagggg gacgcggcgg gcgctgcgcc gggcgagcgc gaagagctgc 540
tgctgctgca gagcacggcc gagcagctgc gccagacggc gctgcaagca ggaggcgcgc 600
atccgcgccg accaggacac catccgtgag ctcaccggca agctgggccg ctgcgagagc 660
ggcctgccgc gcggcctcca gggcgccggg ccccgccgcg acaccatggc cgacggcccc 720
tgggactcgc ctgcgctcat tctggagctg gaggacgccg tgcgcgccct gcgggaccgc 780
atcgaccgca gcaggagctt ccagcccgtg tgaacctctc agctgcccca gccccagtct 840
ctgctgtgcc caccggccta cactccaaga tggaccagct ggaggggcag ctgctggccc 900
aggtgctggc actggagaag gagcgtgtgg ccctcagcca cagcagccgc cggcagaggc 960
aggaagtgga aaaggagttg gacgtcctgc agggtcgtgt ggctgagctg gagcacgggt 1020
cctcagccta cagtcctcca gatgccttca agatcagcat ccccatccgt aacaactaca 1080
tgtacgcccg cgtgcggaag gctctgcccg agctctacgc attcaccgcc tgcatgtggc 1140
tgcggtccag gtccagcggc accggccagg gcaccccctt ctcctactca gtgcccgggc 1200
aggccaacga gattgtactg ctagaggcgg gccatgagcc catggagctg ctgatcaacg 1260
acaaggtggc ccagctgccc ctgagcctga aggacaatgg ctggcaccac atctgcatcg 1320
cctggaccac aagggatggc ctatggtctg cctaccagga cggggagctg cagggctccg 1380
gtgagaacct ggctgcctgg caccccatca agcctcatgg gatccttatc ttgggccagg 1440
agcaggatac cctgggtggc cggtttgatg ccacccaggc ctttgtcggt gacattgccc 1500
agtttaacct gtgggaccac gccctgacac cagcccaggt cctgggcatt gccaactgca 1560
ctgcgccact gctgggcaac gtccttccct gggaagacaa gttggtggag gcctttgggg 1620
gtgcaacaaa ggctgccttc gatgtctgca aggggagggc caaggcatga ggggccacct 1680
catccagggc ccctcccttg cctgccactt tggggacttg aggggggtca tattccctcc 1740
tcagcctgcc cacgcactgg ccttccctcc tgccccactc ctggctgtgc ctcccatttc 1800
ccctcacctg tacccacacc tccagaatgc cctgccctgc gagtgtgtcc cct 1853

<210> 3
<211> 12
<212> PRT
<213> Artifical Sequence

<220>
<223> Description: Antigenic Peptide

<400> 3

Cys Leu Asp Ala Gly Pro Gly Glu Ala Arg Ser Gly 12

<210> 4
<211> 15
<212> PRT
<213> Artifical Sequence

<220>
<223> Description: Antigenic Peptide

<400> 4

Cys Ser Gly Ala Glu Gln Gly Asp Ala Ala Gly Glu Arg Ala Glu 15

<210> 5
<211> 34
<212> DNA
<213> Artifical Sequence

<220>
<223> Description: Primer

<400> 5

atcgaattca tgccggccgg ccgcgcgcgc acct                                34

<210> 6
<211> 21
<212> DNA
<213> Artifical Sequence

<220>
<223> Description: Primer

<400> 6

aggccaacga gctggtcctc a                                              21

<210> 7
<211> 19
<212> DNA
<213> Artifical Sequence

<220>
<223> Description: Primer

<400> 7

tgggattcag cccaggcga                                                 19

<210> 8
<211> 48
<212> DNA
<213> Artifical Sequence

<220>
<223> Description: Primer

<400> 8

cgataagctt gagagaagag acgcacaaac agatcatcgc cgcacaag                 48

<210> 9
<211> 27
<212> DNA
<213> Artifical Sequence

<220>
<223> Description: Primer

<400> 9

tgtgcgtgtg cagggcgacg gcctcgg                                        27

<210> 10
<211> 33
<212> DNA
<213> Artifical Sequence

<220>

<223> Description: Primer

<400> 10

atcgaattcc tgaagttcct ggccgtgctg ctg                                    33



<210> 11
<211> 30
<212> DNA
<213> Artifical Sequence

<220>
<223> Description: Primer

<400> 11

ccggcagagg caggaagtgg aaaaggagtt                                        30



<210> 12
<211> 24
<212> DNA
<213> Artifical Sequence

<220>
<223> Description: Primer

<400> 12

aaggcatctg gaggactgta ggct                                              24



<210> 13
<211> 25
<212> DNA
<213> Artifical Sequence

<220>
<223> Description: Primer

<400> 13

gcgtgggctg gctgaggagg gaata                                             25



<210> 14
<211> 21
<212> DNA
<213> Artifical Sequence

<220>
<223> Description: Primer

<400> 14

tcgcagggca gggcattctg g                                                 21



<210> 15
<211> 21
<212> DNA

<213> Artifical Sequence

<220>
<223> Description: Primer

<400> 15

aggccaacga gctggtcctc a                                                21

<210> 16
<211> 19
<212> DNA
<213> Artifical Sequence

<220>
<223> Description: Primer

<400> 16

tgggattcag cccaggcga                                                   19

<210> 17
<211> 26
<212> DNA
<213> Artifical Sequence

<220>
<223> Description: Primer

<400> 17

gcctctacca gcttgtcttc ccaggg                                           26

<210> 18
<211> 21
<212> DNA
<213> Artifical Sequence

<220>
<223> Description: Primer

<400> 18

cgatatcgct gcgctcgtcg t                                                21

<210> 19
<211> 20
<212> DNA
<213> Artifical Sequence

<220>
<223> Description: Primer

<400> 19

atctccttct gcatcctgtc                                                  20

<210> 20
<211> 21
<212> DNA
<213> Artifical Sequence

<220>
<223> Description: Primer

<400> 20

gcgtgcggcg cggccggcca g                                                21

<210> 21
<211> 21
<212> DNA
<213> Artifical Sequence

<220>
<223> Description: Primer

<400> 21

ctggccggcc gcgccgcacg c                                                21

## Description

The present invention relates to nucleic acid sequences encoding the human Pentraxin receptor and related proteins as well as to a pharmaceutical composition comprising a therapeutically effective amount of (a) Pentraxin I or the human Pentraxin receptor, (b) a nonfunctional variant of the protein of (a), (c) an antibody to the protein of (a) or (b), (d) a nucleic acid sequence encoding the protein of (a) or (b), (e) an antisense RNA sequence characterized in that it is complementary to a Pentraxin I or human Pentraxin receptor mRNA and can selectively bind to said mRNA, said sequence being capable of inhibiting the synthesis of Pentraxin I or the human Pentraxin receptor, or (f) a ribozyme characterized in that it is complementary to a Pentraxin I or human Pentraxin receptor mRNA and can selectively bind to and cleave said mRNA, thus inhibiting the synthesis of Pentraxin I or the human Pentraxin receptor. Moreover, the present invention relates to the use of the above compounds for treating neuronal disorders or providing a neuroprotective effect. Finally, the above compounds are useful for detecting a disease associated with a neuronal disorder based on a deregulated expression of the above proteins or the presence of Pentraxin I/human Pentraxin receptor having no or at least reduced biological activity.

Pentraxins are a family of proteins that include neuronal Pentraxin I (Ptx I), neuronal Pentraxin II (Ptx II) and neuronal Pentraxin Receptor (Ptx R) as well as other non-neuronal members such as serum amyloid P protein (SAP), C-reactive protein (CRP) and Pentraxin III which are cytokine-inducible acute phase proteins implicated in the immune response whose concentrations in the blood increase after an infection or a trauma event. The proteins of the pentraxin family are characterised by their ability to form pentameric complexes and have been proposed to mediate the uptake of bacteria, toxins and extracellular debris in a calcium-dependent manner.

Pentraxin I was identified as a binding protein for the snake venom toxin taipoxin. mRNA of rat and human Pentraxin I has a large size (5.07 kb), is localised in the nervous system and contains a signal peptide for extracellular export. It was suggested that Pentraxin I plays a role in removing synaptic debris and in neuronal plasticity. Rat neuronal pentraxin receptor has been identified through its binding to the snake toxin taipoxin and to other neuronal pentraxins. Rat pentraxin receptor contains a putative N-terminal transmembrane domain, suggesting that it can act as a receptor for different members of this family. Pentraxin II, also called NARP (neuronal activity-regulated pentraxin) was described as an immediate-early gene that is rapidly induced in neurons of the hippocampus and cortex after synaptic activity. Pentraxin II seems to play a role in the modification of cellular properties related to long-term neuroplasticity. Unfortunately, the exact physiological role of the members of the pentraxin family is unknown at the moment. On the other hand, so far the knowledge of compounds useful for treating neuronal diseases such as a stroke, an acute head trauma, multiple sclerosis and a spinal cord injury, or for providing neuroprotective effects is rather limited.

Thus, the technical problem underlying the present invention is to provide means for treating diseases associated with a neuronal disorder or for providing a neuroprotective effect.

The solution to the above technical problem has been achieved by providing the embodiments characterized in the claims. It could be shown that Pentraxin I and Pentraxin receptor play a crucial role in neurotoxicity and that the repression of the expression of Pentraxin I and Pentraxin receptor, respectively, has a neuroprotective effect.

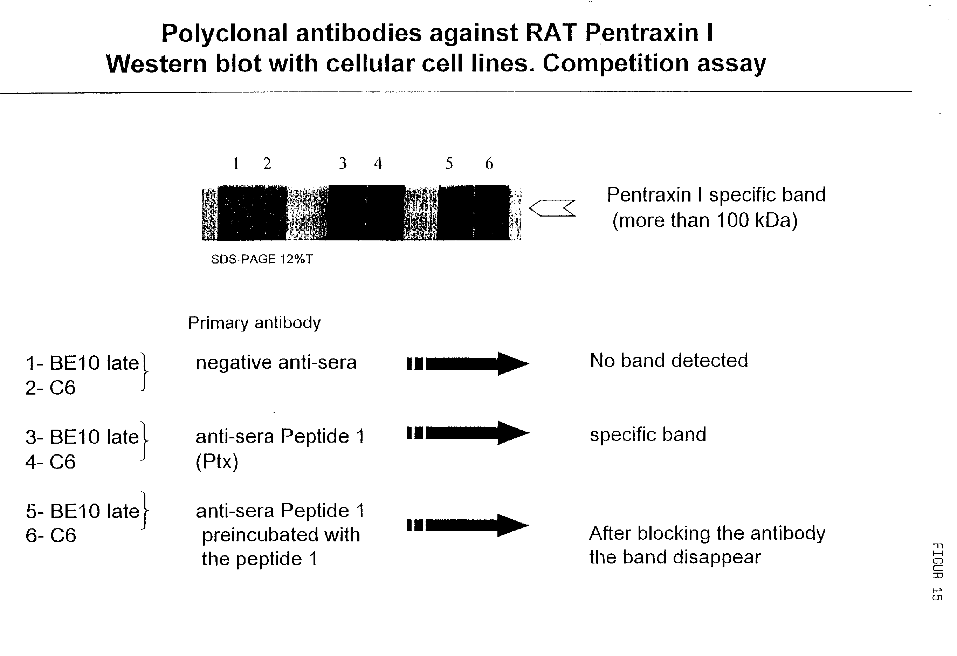

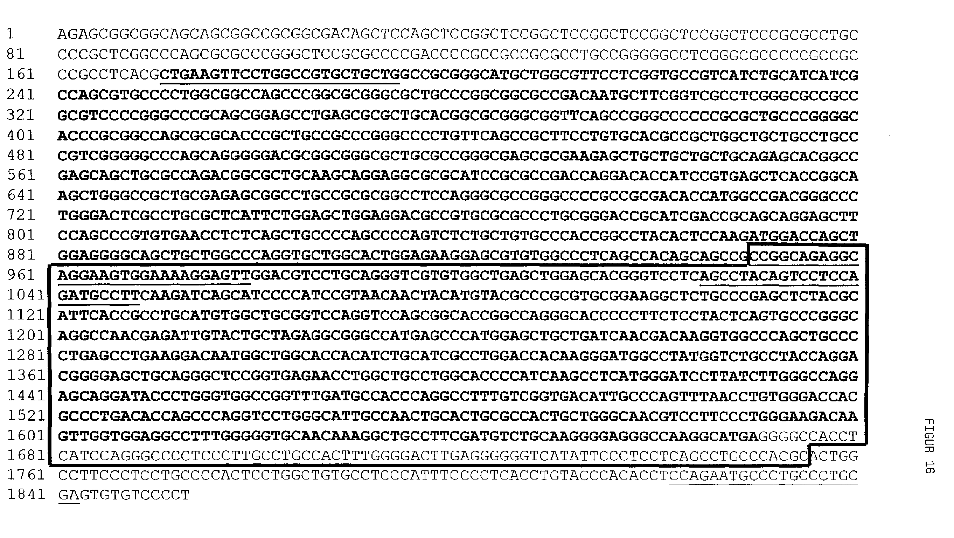

Thus, in one aspect, the present invention relates a nucleic acid sequence encoding the human Pentraxin receptor or a protein exhibiting biological properties of the human Pentraxin receptor and being selected from the group consisting of
(a) a nucleic acid sequence comprising the coding region of the nucleotide sequence depicted in Figure 16;
(b) a nucleic acid sequence which hybridizes to a nucleic acid sequence specified in (a);
(c) a nucleic acid sequence which deviates from the nucleic acid sequences specified in (a) and (b) due to the degeneracy of the genetic code; and
(d) a nucleic acid sequence, which represents a fragment, derivative or allelic variation of a nucleic acid sequence specified in (a) to (c).

As used herein, a protein exhibiting biological properties of the human Pentraxin receptor is understood to be a protein having at least one of the activities of the human Pentraxin receptor as illustrated in the Examples, below.

In a first embodiment, the invention provides a nucleic acid sequence encoding the human Pentraxin receptor comprising the coding region of the nucleotide sequence depicted in Figure 16.

The present invention provides not only the nucleic acid sequence identified in Figure 16, but also a sample of plasmid DNA containing a human Pentraxin receptor cDNA. The nucleotide sequence of the deposited clone can readily be determined by sequencing the deposited clone in accordance with known methods. The predicted amino acid sequence can then be verified from such deposits. Moreover, the amino acid sequence of the protein encoded by the deposited clone can also be directly determined by peptide sequencing or by expressing the protein in a suitable host cell containing the deposited human Pentraxin receptor encoding cDNA, collecting the protein, and determining its sequence.

The nucleic acid sequences of the invention can be both DNA and RNA molecules. Suitable DNA molecules are, for example, genomic or cDNA molecules. It is understood that all nucleic acid molecules encoding all or a portion of human Pentraxin receptor are also included, as long as they encode a polypeptide with human Pentraxin receptor activity. The nucleic acid sequences of the invention can be isolated from natural sources or can be synthesized according to known methods.

The present invention also provides nucleic acid sequences which hybridize to the above nucleic acid sequences. As used herein, the term "hybridize" has the meaning of hybridization under conventional hybridization conditions, preferably under stringent conditions as described, for example, in Sambrook et al., Molecular Cloning, A Laboratory Manual, 2^{nd} edition (1989) Cold Spring Harbor Laboratory Press, Cold Spring Harbor, NY. Also contemplated are nucleic acid sequences that hybridize to the human Pentraxin receptor nucleic acid sequences at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 mg/ml salmon sperm blocking DNA, followed by washes at 50°C with 1 X SSPE, 0.1% SDS. In addition, to achieve even lower stringency, washes performed following stringent hybridization can be done at higher salt concentrations (e.g. 5X SSC). Variations in the above conditions may be accomplished through the inclusion and/or substitution of alternate blocking reagents used to suppress background in hybridization experiments. The inclusion of specific blocking reagents may require modification of the hybridization conditions described above, due to problems with compatibility.

Nucleic acid sequences that hybridize to the nucleic acid sequences of the invention can be isolated, e.g., from genomic or cDNA libraries that were produced from rat or human cell lines or tissues. In order to identify and isolate such nucleic acid molecules the molecules of the invention or parts of these molecules or the reverse complements of these molecules can be used, for example by means of hybridization according to conventional methods (see, e.g., Sambrook et al., supra). As a hybridization probe nucleic acid molecules can be used, for example, that have exactly or basically the nucleotide sequence depicted in Figure 16 or parts of these sequences. The fragments used as hybridization probe can be synthetic fragments that were produced by means of conventional synthesis methods and the sequence of which basically corresponds to the sequence of a nucleic acid molecule of the invention.

The nucleic acid sequences of the present invention also include molecules with sequences that are degenerate as a result of the genetic code.

In a further embodiment, the present invention provides nucleic acid sequences which comprise fragments, derivatives and allelic variants of the nucleic acid sequences described above encoding a protein of the invention. "Fragments" are understood to be parts of the nucleic acid sequences that are long enough to encode one of the described proteins. The term "derivative" in this context means that the sequences of these molecules differ from the sequences of the nucleic acid sequences described above at one or several positions but have a high level of homology to these sequences. Homology hereby means a sequence identity of at least 40 %, in particular an identity of at least 60 %, preferably of more than 80 % and particularly preferred of more than 90 %. These proteins encoded by the nucleic acid sequences have a sequence identity to the amino acid sequence encoded by the nucleic acid sequence depicted in Figure 16 of at least 80 %, preferably of 85 % and particularly preferred of more than 90 %, 95 %, 97 % and 99 %. The deviations to the above-described nucleic acid sequences may have been produced by deletion, substitution, insertion or recombination.

The nucleic acid sequences that are homologous to the above-described sequences and that represent derivatives of these sequences usually are variations of these sequences that represent modifications having the same biological function. They can be naturally occurring variations, for example sequences from other organisms, or mutations that can either occur naturally or that have been introduced by specific mutagenesis. Furthermore, the variations can be synthetically produced sequences. The allelic variants can be either naturally occurring variants or synthetically produced variants or variants produced by recombinant DNA processes.

Generally, by means of conventional molecular biological processes it is possible (see, e.g., Sambrook et al., supra) to introduce different mutations into the nucleic acid molecules of the invention. As a result human Pentraxin receptor proteins or related proteins with possibly modified biological properties are synthesized. One possibility is the production of deletion mutants in which nucleic acid molecules are produced by continuous deletions from the 5'- or 3'-terminal of the coding DNA sequence and that lead to the synthesis of proteins that are shortened accordingly. Another possibility is the introduction of single-point mutations at positions where a modification of the amino acid sequence influences, e.g., the enzyme activity or the regulation of the enzyme. By this method muteins can be produced, for example, that possess a modified Kₘ-value or that are no longer subject to the regulation mechanisms that normally exist in the cell, e.g. with regard to allosteric regulation or covalent modification. Such muteins might also be valuable as therapeutically useful antagonists of human Pentraxin receptor.

For the manipulation in prokaryotic cells by means of genetic engineering the nucleic acid sequences of the invention or parts of these sequences can be introduced into plasmids allowing a mutagenesis or a modification of a sequence by recombination of DNA sequences. By means of conventional methods (see e.g. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The proteins encoded by the various variants of the nucleic acid sequences of the invention show certain common characteristics, such as enzyme activity, molecular weight, immunological reactivity or conformation or physical properties like the electrophoretical mobility, chromatographic behavior, sedimentation coefficients, solubility, spectroscopic properties, stability; pH optimum, temperature optimum.

The invention furthermore relates to vectors containing the nucleic acid sequences of the invention. Preferably, they are plasmids, cosmids, viruses, bacteriophages and other vectors usually used in the field of genetic engineering. Vectors suitable for use in the present invention include, but are not limited to the T7-based expression vector for expression in bacteria, the pMSXND expression vector for expression in mammalian cells and baculovirus-derived vectors for expression in insect cells. Preferably, the nucleic acid sequence of the invention is operatively linked to the regulatory elements in the recombinant vector of the invention that guarantee the transcription and synthesis of an RNA in prokaryotic and/or eukaryotic cells that can be translated. The nucleotide sequence to be transcribed can be operatively linked to a promoter like a T7, metallothionein I or polyhedrin promoter.

In a further embodiment, the present invention relates to recombinant host cells transiently or stably containing the nucleic acid sequences or vectors of the invention. A host cell is understood to be an organism that is capable to take up *in vitro* recombinant DNA and, if the case may be, to synthesize the proteins encoded by the nucleic acid molecules of the invention. Preferably, these cells are prokaryotic or eukaryotic cells, for example mammalian cells, bacterial cells, insect cells or yeast cells. The host cells of the invention are preferably characterized by the fact that the introduced nucleic acid sequence of the invention either is heterologous with regard to the transformed cell, i.e. that it does not naturally occur in these cells, or is localized at a place in the genome different from that of the corresponding naturally occurring sequence.

A further embodiment of the invention relates to human Pentraxin receptor or proteins exhibiting biological properties of the human Pentraxin receptor and being encoded by the nucleic acid sequences of the invention, as well as to methods for their production, whereby, e.g, a host cell of the invention is cultivated under conditions allowing the synthesis of the protein and the protein is subsequently isolated from the cultivated cells and/or the culture medium. Isolation and purification of the recombinantly produced proteins may be carried out by conventional means including preparative chromatography and affinity and immunological separations involving affinity chromatography with monoclonal or polyclonal antibodies, e.g. with the antibody described in the Examples, below.

The proteins of the invention not only comprise recombinantly produced proteins but include isolated naturally occurring proteins, synthetically produced proteins, or proteins produced by a combination of these methods. Means for preparing such proteins are well understood in the art.

In a further aspect, the present invention relates to a pharmaceutical composition comprising a therapeutically effective amount of at least one of the following compounds:
(a) Pentraxin I or the human Pentraxin receptor encoded by the nucleic acid sequences of the invention;
(b) a nonfunctional variant of the protein of (a);
(c) an antibody to the protein of (a) or (b);
(d) a nucleic acid sequence encoding the protein of (a) or (b);
optionally in combination with a pharmaceutically acceptable carrier.

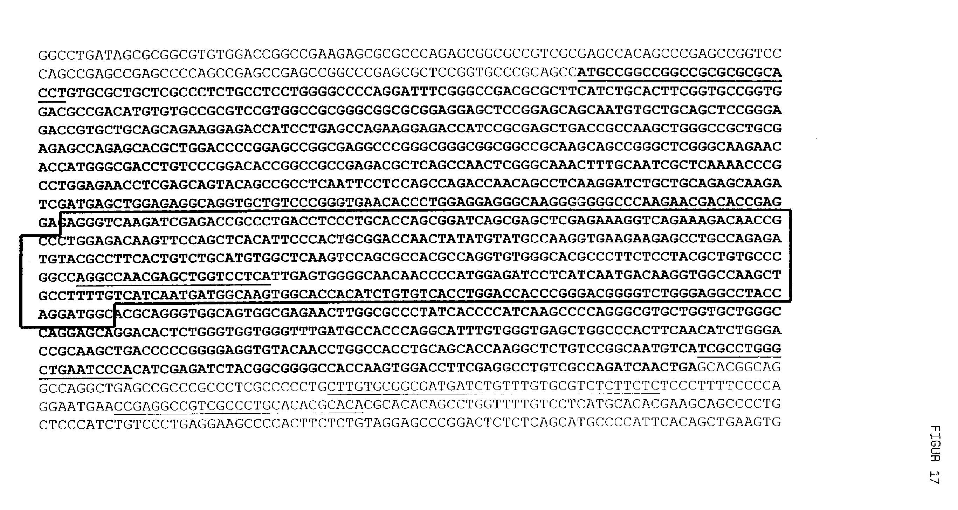

As used herein, the term "Pentraxin I" refers to a protein or polypeptide being encoded by the nucleotide sequence as described in Perin et al., Genomics 36, 1996, 543-545, the sequence of the GeneBank database accession no. U61849 or the nucleotide sequence shown in Figure 17, including Pentraxin I variants containing mutations or deletions which do not substantially affect the activity thereof. Such mutations include substitutions of one or more amino acids, particularly by homologues thereof, as well as additions of one or more amino acids, especially at the N or C termini. Deletions include deletions from the N or C termini. Substitutions by both naturally-occurring and synthetic amino acids are possible. Also included in this definition are Pentraxin I variants modified by chemical modification or enzymatic modification. Further, fragments of Pentraxin I, whether chemically synthesized or produced by a biological method, whether modified or unmodified, are included within the definition of the term "Pentraxin I".

As used herein, the term "a nonfunctional variant of the protein of (a)" refers to a Pentraxin I variant or a human Pentraxin receptor variant which exhibits a substantial loss of biological activity or a complete loss of biological activity. Such a variant may contain the modifications as described above for the human Pentraxin receptor or with respect to the definition of the term "Pentraxin I", except that these modifications result in the generation of a "nonfunctional" Pentraxin I and human Pentraxin receptor. Such a variant might be useful as an antagonist of the native protein.

As used herein, the term "an antibody to the protein of (a) or (b)" relates to polyclonal or monoclonal antibodies, preferably, relates to antibodies which consist essentially of pooled monoclonal antibodies with different epitopic specificities, as well as distinct monoclonal antibody preparations. Monoclonal antibodies are made from an antigen containing fragments of Pentraxin I, the human Pentraxin receptor or the variants described above by methods well known to those skilled in the art (see, e.g., Kohler and Milstein, Nature 2556, 1975, 495-497). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. Thus, these fragments are preferred, as well as the products of a Fab or other immunoglobulin expression library. Moreover, antibodies of the present invention include chimeric, single chain, and humanized antibodies.

As used herein the term "a nucleic acid sequence encoding the protein of (a) or (b)" refers to the nucleotide sequence described by Perin et al., supra, the GenBank sequence with the accession no. U61849 or the nucleotide sequence depicted in Figure 17 as regards Pentraxin I and to the nucleic acid sequences of the invention described above as regards the human Pentraxin receptor, e.g. the nucleic acid sequence shown in Figure 16. These nucleic acid sequences can be both DNA and RNA molecules. Suitable DNA molecules are, for example, genomic or cDNA molecules. It is understood that the nucleic acid sequences encoding all or a portion of Pentraxin I additionally comprise sequences as defined for the human Pentraxin receptor coding nucleic acid sequences, e.g. hybridizing sequences, fragments, derivatives, allelic variations etc.. Finally, the above definition also includes nucleic acid sequence encoding a nonfunctional Pentraxin I variant or nonfunctional human Pentraxin receptor variant which is, e.g., useful for "knocking out" the gene encoding the biologically active natural protein.

In a further preferred embodiment, the present invention relates to a pharmaceutical composition comprising (a) an antisense RNA sequence characterized in that it is complementary to an mRNA transcribed from a gene encoding Pentraxin I or the nucleic acid sequences of the invention encoding the human Pentraxin receptor or a protein exhibiting the biological activity of the human Pentraxin receptor or a part thereof and can selectively bind to said mRNA or part thereof, said sequence being capable of inhibiting the synthesis of Pentraxin I or the human Pentraxin receptor or a protein having the biological properties of the human Pentraxin receptor, or (b) a ribozyme characterized in that it is complementary to an mRNA transcribed from a gene encoding Pentraxin I or the nucleic acid sequences of the invention encoding the human Pentraxin receptor or a protein exhibiting the biological properties of the human Pentraxin receptor or a part thereof and can selectively bind to and cleave said mRNA or part thereof, thus inhibiting the synthesis of Pentraxin I or the human Pentraxin receptor or a protein having the biological properties of the human Pentraxin receptor.

Ribozymes which are composed of a single RNA chain are RNA enzymes, i.e. catalytic RNAs, which can intermolecularly cleave a target RNA, for example the mRNA transcribed from the Pentraxin I gene. It is now possible to construct ribozymes which are able to cleave the target RNA at a specific site by following the strategies described in the literature. (see, e.g., Tanner et al., Antisense Research and Applications, CRC Press Inc. 1993, 415-426). The two main requirements for such ribozymes are the catalytic domain and regions which are complementary to the target RNA and which allow them to bind to its substrate, which is a prerequisite for cleavage. Said complementary sequences, i.e., the antisense RNA or ribozyme, are useful for repression of Pentraxin I expression, e.g. for providing a neuroprotective effect. Preferably, the antisense RNAs and ribozymes of the invention are complementary to the coding region of the Pentraxin I/human Pentraxin receptor mRNA, e.g. to the 5' part of the coding region. The person skilled in the art provided with the Pentraxin I/human Pentraxin receptor encoding nucleic acid sequences will be in a position to produce and utilize the above described antisense RNAs or ribozymes. The region of the antisense RNA and ribozyme, respectively, which shows complementarity to the Pentraxin I/human Pentraxin receptor mRNA preferably has a length of at least 15, in particular of at least 25 nucleotides and particularly preferred is a length of at least 50 nucleotides.

In still a further embodiment, the present invention relates to inhibitors which fulfill a similar purpose as the antisense RNAs or ribozymes mentioned above, i.e. reduction or elimination of biologically active Pentraxin I molecules or human Pentraxin receptor molecules. Such inhibitors can be, for instance, structural analogues of the natural protein that act as antagonists. In addition, such inhibitors comprise molecules identified by the use of the recombinantly produced Pentraxin I or human Pentraxin receptor, e.g. the recombinantly produced protein can be used to screen for and identify inhibitors, for example, by exploiting the capability of potential inhibitors to bind to the protein under appropriate conditions. The inhibitors can, for example, be identified by preparing a test mixture wherein the inhibitor candidate is incubated with Pentraxin I or the human Pentraxin receptor under appropriate conditions that allow Pentraxin I or the human Pentraxin receptor to be in a native conformation. Such an in vitro test system can be established according to methods well known in the art. Inhibitors can be identified, for example, by first screening for either synthetic or naturally occuring molecules that bind to the recombinantly produced protein and then, in a second step, by testing those selected molecules in cellular assays for inhibition of Pentraxin I or the human Pentraxin receptor, as reflected by inhibition of at least one of the biological activities of said proteins as described in the Examples, below. Such screening for molecules that bind Pentraxin I or the human Pentraxin receptor could easily performed on a large scale, e.g. by screening candidate molecules from libraries of synthetic and/or natural molecules. Such an inhibitor is, e.g. a synthetic organic chemical, a natural fermentation product, a substance extracted from a microorganism, plant or animal, or a peptide.

In another preferred embodiment, the present invention relates to the use of one of the above disclosed compounds for the preparation of a pharmaceutical composition for preventing, treating or ameliorating a disease associated with a neuronal disorder or for providing a neuroprotective effect. Examples of such a disease are a stroke (brain ischemia), an acute head trauma, multiple sclerosis, a spinal cord injury and Alzheimer disease. Furthermore, the above compounds can be used as proapoptotic proteins. Particularly by overpression or upregulation of Pentraxin I, brain tumors can be treated.

For administration the above compounds are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, general health and other drugs being administered concurrently.

The delivery of the above described nucleic acid sequences, i.e. antisense RNAs or ribozymes, can be achieved by direct application or, preferably, by using a recombinant expression vector such as a chimeric virus containing these compounds or a colloidal diserpersion system. By delivering these nucleic acids to the desired target, e.g., the intracellular expression of Pentraxin I and, thus, the level of Pentraxin I can be decreased resulting in the inhibition of the negative effects of Pentraxin I discussed above. The above nucleic acids can be administered directly to the target site, e.g., by ballistic delivery, as a colloidal dispersion system or by catheter to a site in artery. The colloidal dispersion systems which can be used for delivery of the above nucleic acids include macromolecule complexes, nanocapsules, microspheres, beads and lipid-based systems including oil-in-water emulsions, (mixed) micelles, liposomes and lipoplexes. The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired site. The targeting of liposomes can be carried out by the person skilled in the art by applying commonly known methods. This targeting includes passive targeting or active targeting (for example by coupling the liposome to a specific ligand, e.g., an antibody, a receptor, sugar, glycolipid, protein etc., by well known methods).

Preferred recombinant vectors useful for gene therapy are viral vectors, e.g. adenovirus, adeno-associated virus (rAAV), herpes virus such as herpes simplex virus type I (HSV I), vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotid encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ the nucleic acid sequences encoding, e.g. an antisense RNA or ribozyme can also be operatively linked to a tissue specific promoter and used for gene therapy. For example, the above described nucleic acid sequences or nucleic acid sequences encoding the antisense RNA or ribozyme can be ligated to brain-specific promoters such as myelin basic protein (MBP) promoter, neuron-specific enolase (NSE) promoter and glial fibrillary acidic protein (GFAP) promoter. Other tissue specific promoters are well known to those skilled in the art.

The above described compounds are particularly useful for preventing, treating or ameliorating a disease such as a stroke, an acute head trauma and brain tumors, e.g. a glioblastoma.

The present invention also relates to a method for detecting a disease associated with a neuronal disorder based on a deregulated expression of Pentraxin I or human Pentraxin receptor or the presence of Pentraxin I or human Pentraxin receptor having no or at least reduced biological activity comprising contacting a target sample suspected to contain Pentraxin I, human Pentraxin receptor or Pentraxin I or human Pentraxin receptor encoding nucleic acid sequences with a reagent which reacts with Pentraxin I or human Pentraxin receptor or the Pentraxin I or the human Pentraxin receptor encoding nucleic acid and detecting abnormal amounts or abnormal forms of Pentraxin I or the human Pentraxin receptor or Pentraxin I or human Pentraxin receptor encoding nucleic acid. When the target is mRNA, the reagent is typically a nucleic acid probe or a primer for PCR. The person skilled in the art is in a position to design suitable nucleic acids probes based on the information as regards the nucleotide sequence of Pentraxin I and human Pentraxin receptor as depicted in Figures 17 and 16, respectively, or the literature disclosed above. When the target is a protein, the reagent is typically an antibody probe (for the definition of the term "antibody probe" see the above definition of the term "antibody"). Detection methods include Northern blot analysis, RNase protection, in situ methods, PCR, LCR, immunoassays and other detection assays that are known to those skilled in the art. The probes can be detectably labeled, for example, with a radioisotope, a bioluminescent compound, a chemiluminescent compound, a fluorescent compound, a metal chelate, or an enzyme.

Expression of Pentraxin I or the human Pentraxin receptor in tissues can be studied with classical immunohistological methods. Other antibody based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium (⁹⁹mTc), and fluorescent labels, such as fluorescein and rhodamine, and biotin. In addition to assaying protein levels in a biological sample, Pentraxin I and the human Pentraxin receptor can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma. A protein-specific antibody or antibody fragment which has been labeled with an appropriate detectable imaging moiety, such as a radioisotope (for example, ¹³¹I, ¹¹²In, ⁹⁹mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ⁹⁹mTc. The labeled antibody will then preferentially accumulate at the location of cells which contain the specific protein. For evaluating whether the concentration of Pentraxin I/human Pentraxin receptor or the Pentraxin I/human Pentraxin receptor encoding mRNA is normal, decreased or increased, thus indicative for a neuronal disorder, the measured concentration is compared with the concentration in a normal tissue. Further diagnostic methods include the determination of the size of the protein or the mRNA, the determination of the nucleotide sequence of the Pentraxin I/human Pentraxin receptor gene by standard sequencing methods etc. and the comparison with the corresponding data obtained from a healthy individual.

For use in the diagnostic research discussed above, kits are also provided by the present invention. Such kits are useful for the detection of a target cellular component, which is Pentraxin I and/or the human Pentraxin receptor or Pentraxin I and/or the human Pentraxin receptor encoding mRNA, wherein the presence of a decreased or increased concentration of the protein or mRNA or abnormal forms of the protein or corresponding nucleic acids (e.g. as regards size, amino acid sequence etc.) are indicative for one of the above described disorders, said kit comprising (a) probe(s) which specifically bind(s) to Pentraxin I and/or the human Pentraxin receptor or Pentraxin I and/or the human Pentraxin receptor encoding nucleic acid sequences, e.g. mRNA. The probe can be detectably labeled. Such probe may be an antibody or oligonucleotide specific for the protein or the corresponding mRNA. In a preferred embodiment, said kit contains an anti-Pentraxin I-antibody and/or an anti-human Pentraxin receptor-antibody and allows said diagnosis by ELISA and contains the antibody bound to a solid support, for example, a polystyrene microtiter dish or nitrocellulose paper, using techniques known in the art. Alternatively, said kits are based on a RIA and contain said antibody marked with a radioactive isotope. In a preferred embodiment of the kit of the invention the antibody is labelled with enzymes, fluorescent compounds, luminescent compounds, ferromagnetic probes or radioactive compounds.

### Brief description of the Figures

- **Figure 1:**: **Differential gene expression in cerebellar granule cell cultures after potassium removal**
- **Figure 2:**: **Glutamate induced neurotoxicity in cerebellar granule cell cultures**
(a) Scheme of glutamate induced neurotoxicity model in cerebellar granule cell cultures, (b) time-course effect of glutamate on the viability of cerebellar granule cells, (c) effect of glucose on the viability under glutamate treatment
- **Figure 3:**: **Differential gene display in rat stroke model**
Three upstream primers (D12, D15, D16) used in combination with a downstream primer (T)₁₁AT for the cDNA amplification are shown on the top of the figure. The specific sequences of each primer are shown on the bottom of the figure. The arrow heads indicate the bands showing differences.
- **Figure 4:**: **Antigenic peptide design for rat Pentraxin I (4/I) and Pentraxin receptor (4/II)**
(I/A) Comparison of the amino acid sequences of rat Pentraxin I and Pentraxin receptor to determine the antigenic specificity.The synthetic peptide for antibody is boxed.
(I/B) Design of peptide 1 based on the sequence of rat Pentraxin I sequence. A cysteine was included in the N-terminus to facilitate the carrier conjugation.
(II/A) Comparison of the amino acid sequences of rat Pentraxin I and Pentraxin receptor to determine the antigenic specificity.The synthetic peptide for antibody is boxed.
(II/B) Design of peptide 2 based on the sequence of rat Pentraxin receptor sequence. A cysteine was included in the N-terminus to facilitate the carrier conjugation.

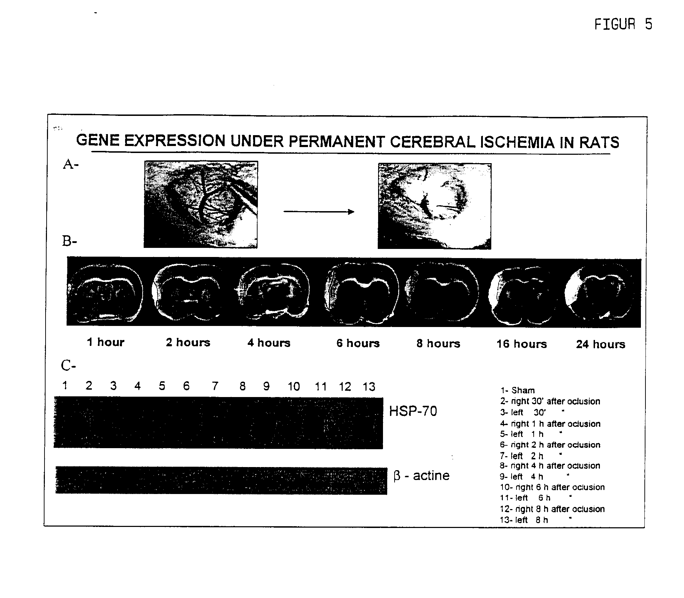

- **Figure 5:**: **Gene expression under permanent cerebral ischemia in rats**
(a) Amplified photography of the diathermy occlusion of the left middle cerebral artery (MCA) in the rat.
b) TTC-stained coronal sections of the injured brains at different times after MCAO (middle central artery occlusion). The infarcted area is not stained (white and light stain).
(c) Levels of actin and HSP-70 (heat shock protein - 70) at both hemispheres at different times after MCAO by northern analysis.

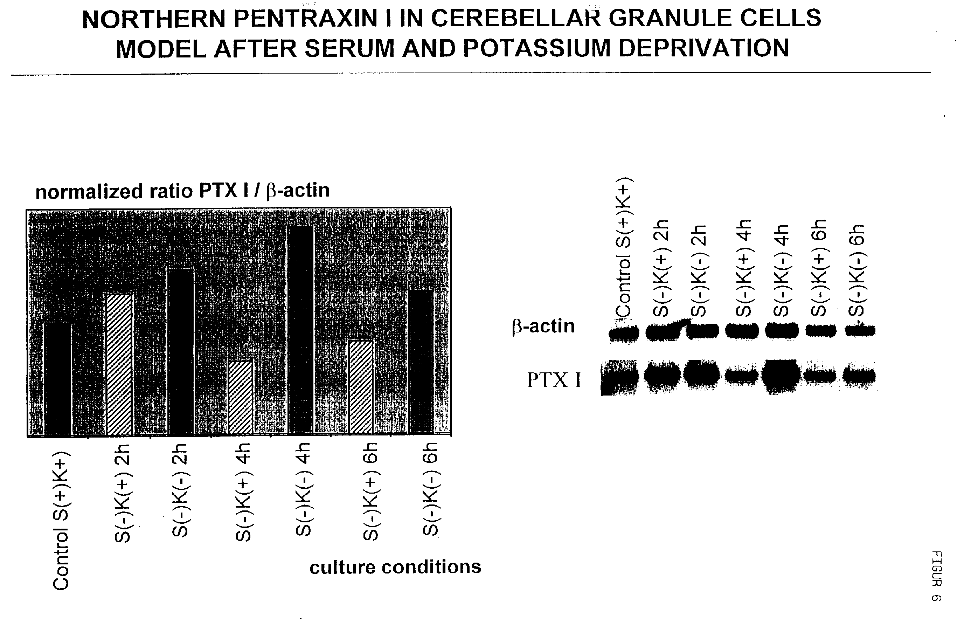

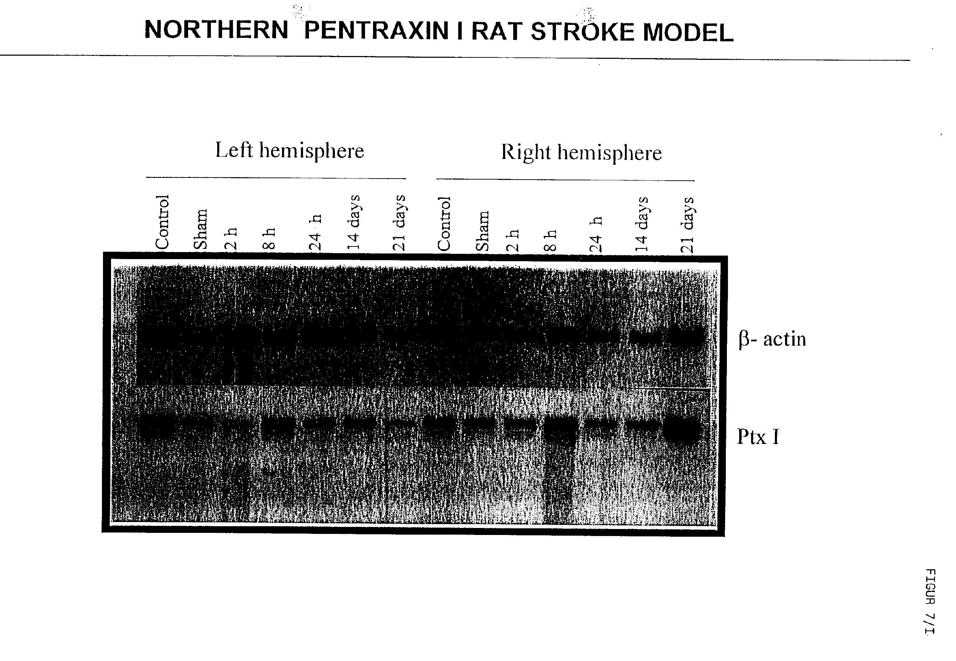

- **Figure 6:**: **Northern analysis of rat Pentraxin I in cerebellar granule cells model after serum and potassium deprivation**
Evaluation of rat Pentraxin I expression and β-actin as a control at different times after treatment.

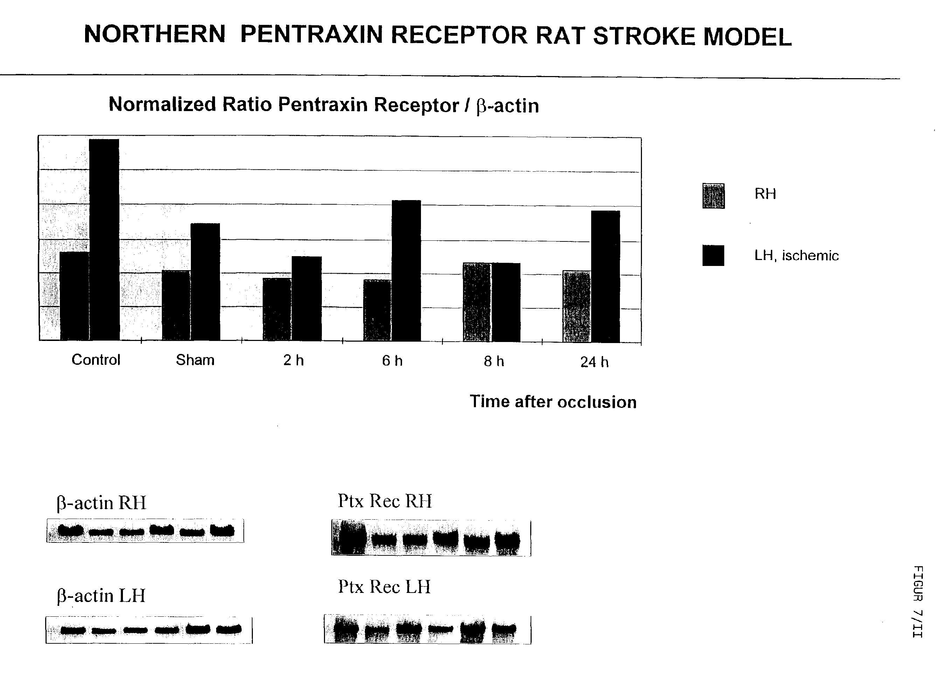

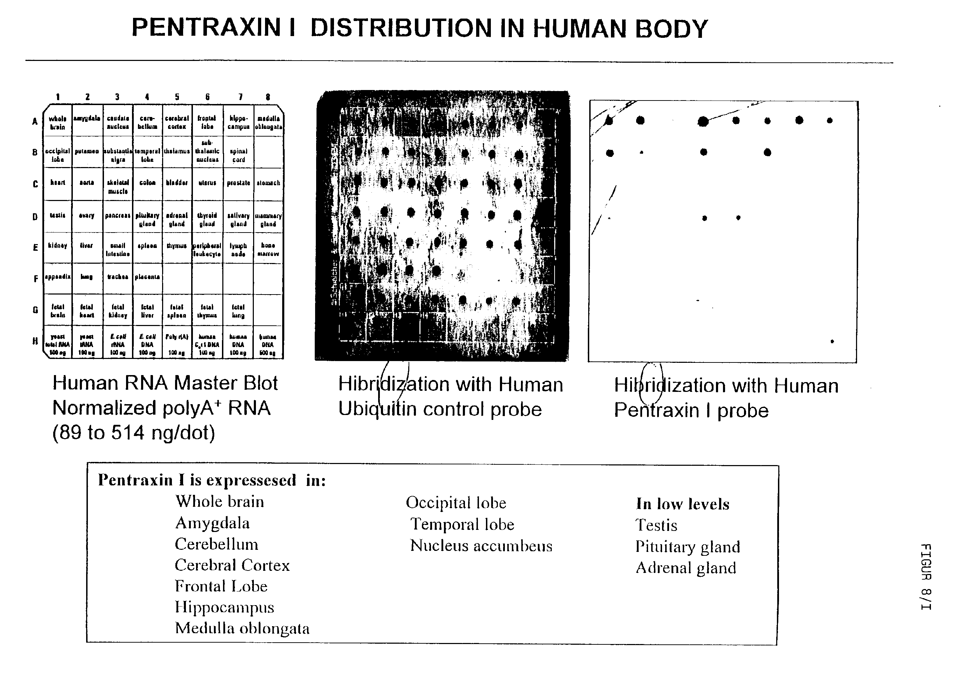

- **Figure 7:**: **Northern analysis of rat Pentraxin I (7/I) and Pentraxin receptor (7/II) in a stroke model**
(7/I) Evaluation of rat Pentraxin I expression and β-actin as a control at different times after MCAO in both hemispheres.
(7/II) Evaluation of rat Pentraxin receptor expression and β-actin as a control at different times after MCAO in both hemispheres.

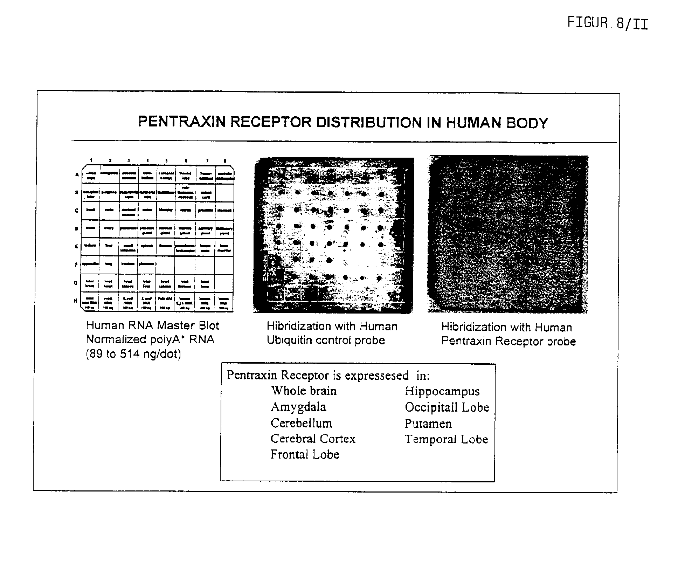

- **Figure 8:**: **Distribution of Pentraxin I (8/I) and Pentraxin receptor (8/II, III) in human body**
Northern blot (Multiple tissue northern blots, Clontech Laboratories Inc, Palo Alto, CA) with Pentraxin I probe (8/I), Pentraxin receptor probe (8/II, III) and ubiquitin as a control (8/I, II).

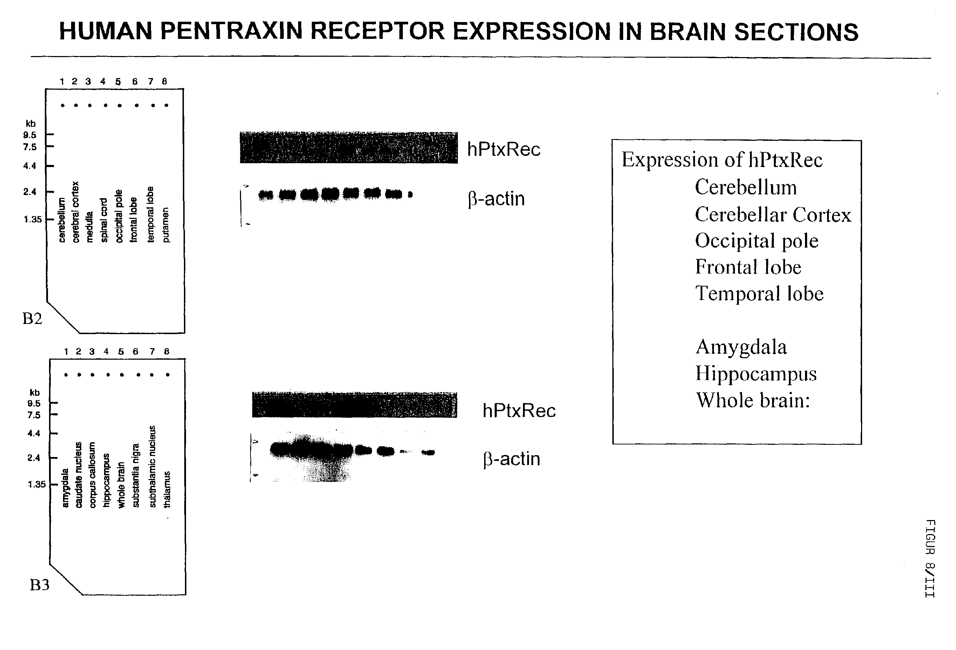

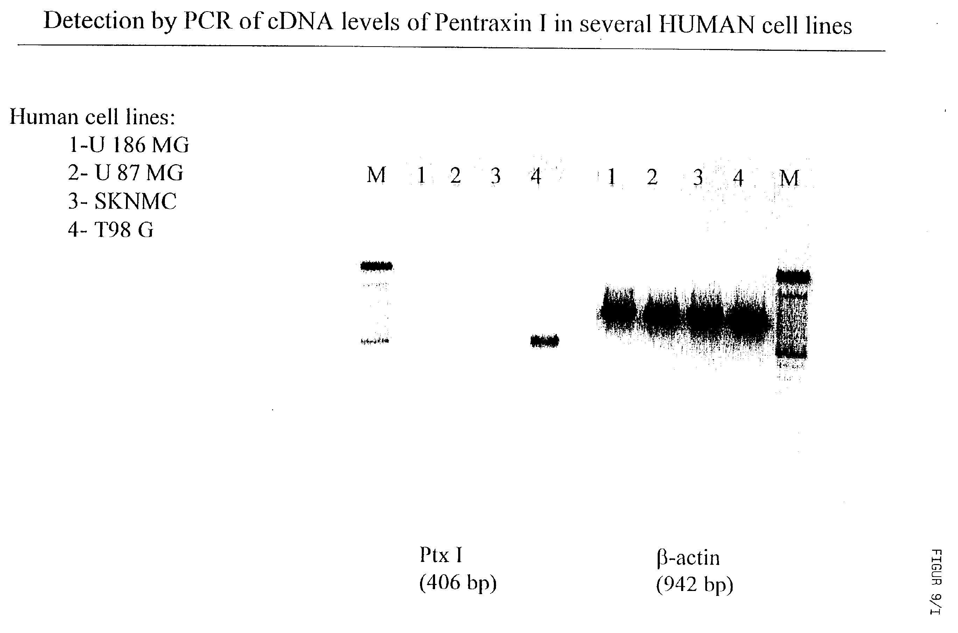

- **Figure 9:**: **Detection by PCR of cDNA levels of Pentraxin I (9/I) and Pentraxin receptor (9/II) in several human cell lines.**

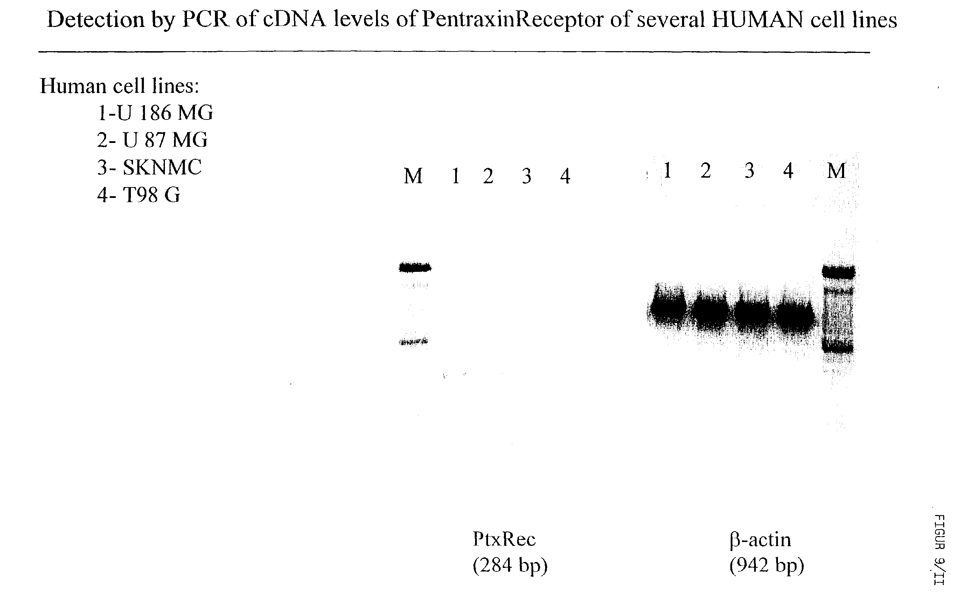

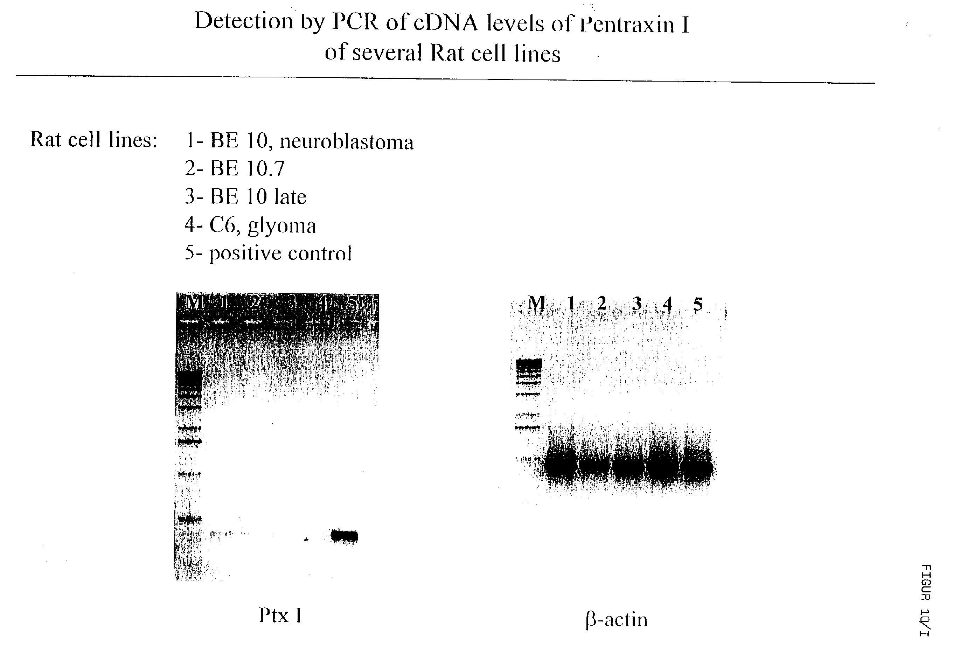

- **Figure 10:**: **Detection by PCR of cDNA levels of Pentraxin I in several rat cell lines (10/I) and Pentraxin receptor in several rat cell lines (10/II).**

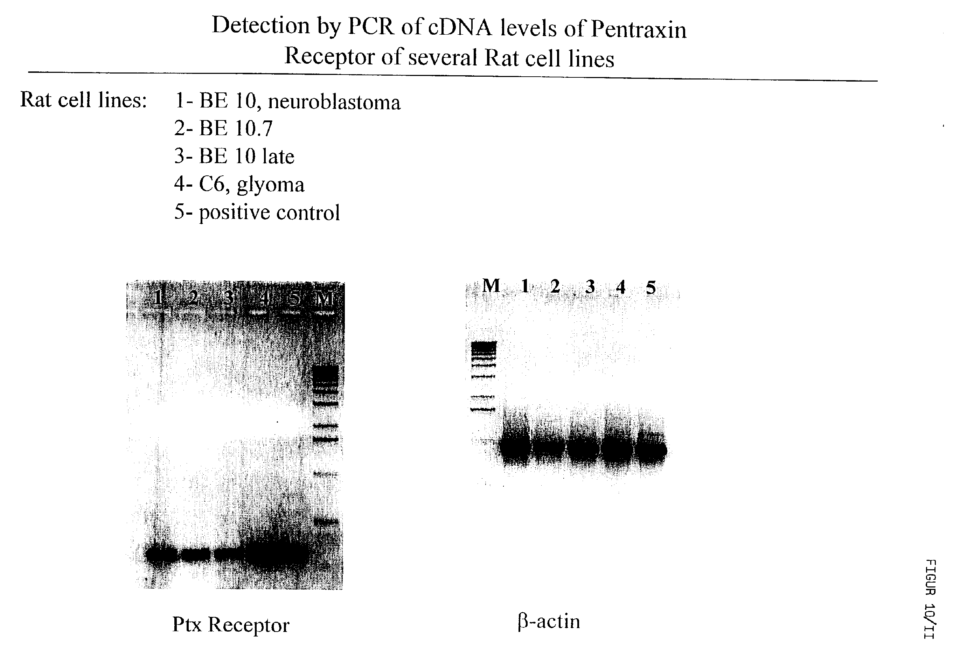

- **Figure 11:**: **Pentraxin I (11/I) and Pentraxin receptor (11/II) expression by semiquantitative PCR at different culture conditions on the glutamate neurotoxicity model in rat cerebellar granule cells.**

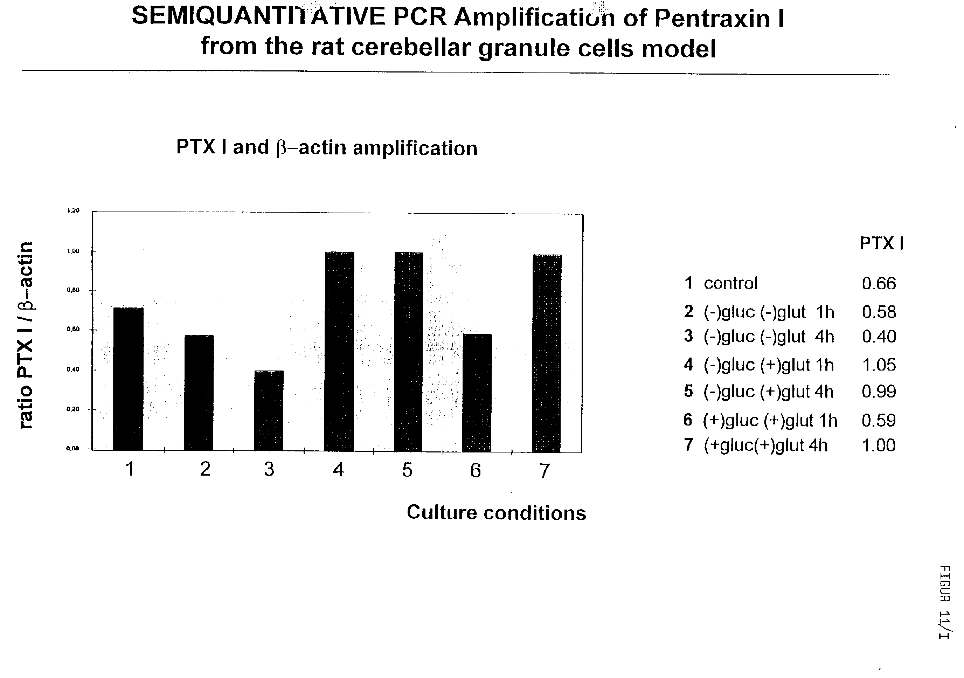

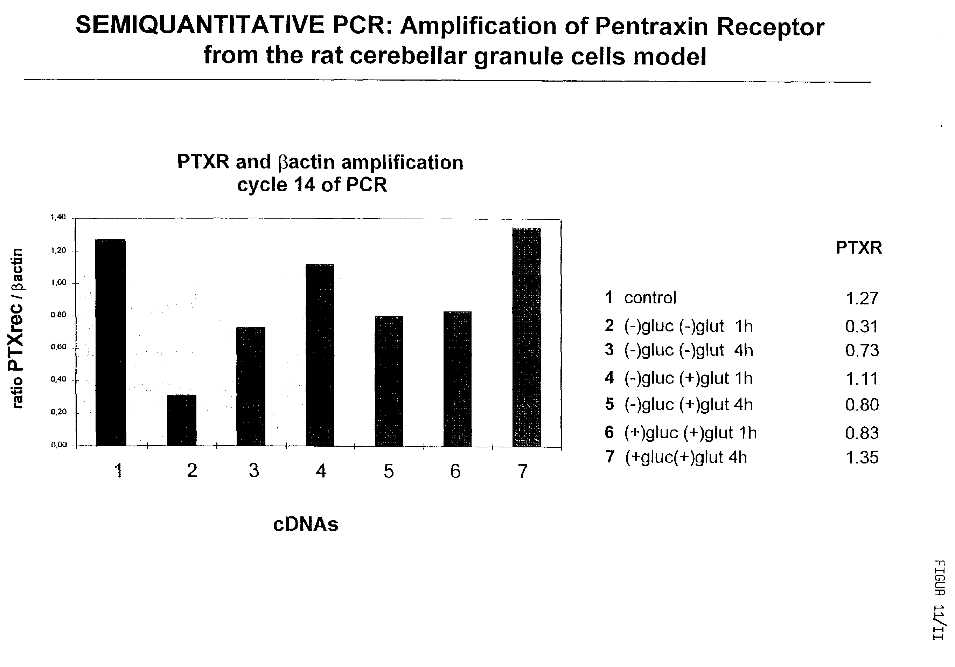

- **Figure 12:**: **Effects of antisense and sense oligonucleotides on low potassium induced neuronal cell death in cerebellar granule cells**

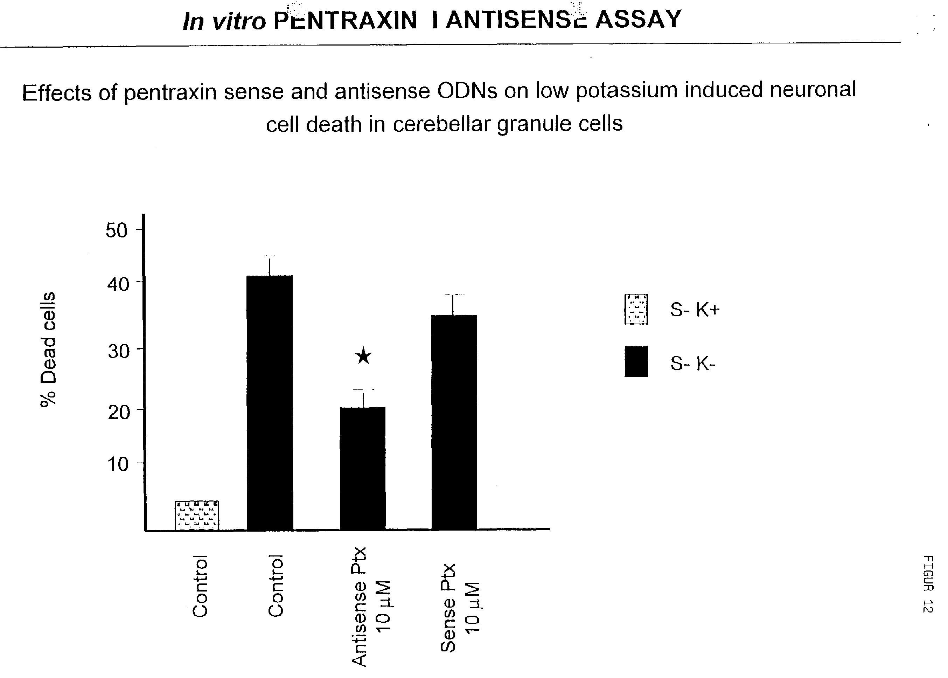

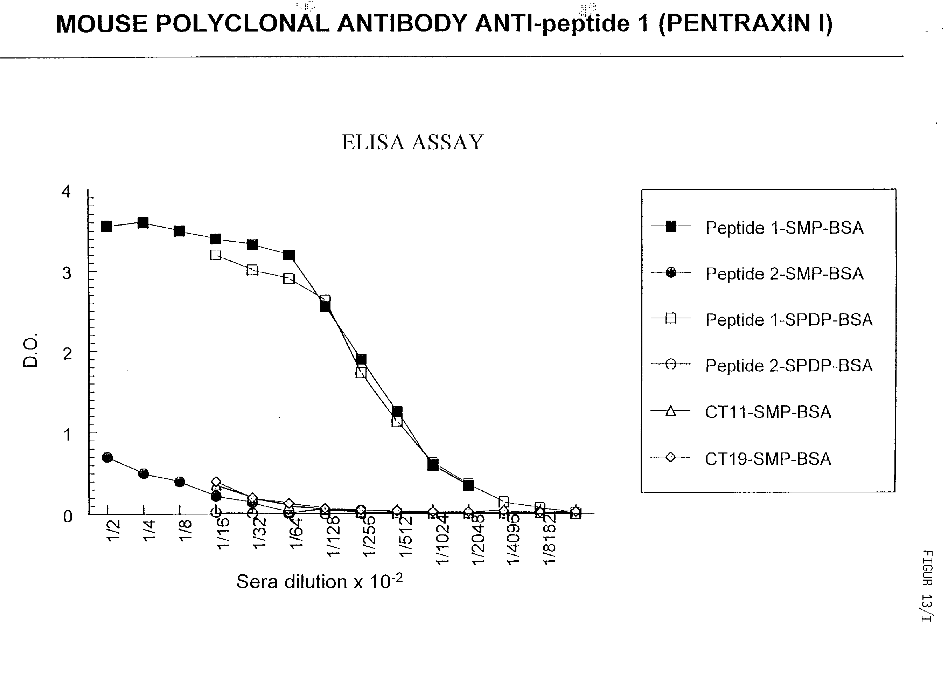

- **Figure 13:**: **ELISA assay for detecting antibodies against rat Pentraxin I and Pentraxin receptor**
The assay was done with peptide 1 (13/I; Pentraxin I) or respectively peptide 2 (13/II; Pentraxin receptor) conjugated with BSA using SMP or SPDP as a linker. In Figure 13/I Peptide 2 (Pentraxin receptor) and CT19 were used as negative controls and in Figure 13/II Peptide 1 (Pentraxin 1) and CT19 were used as negative controls.

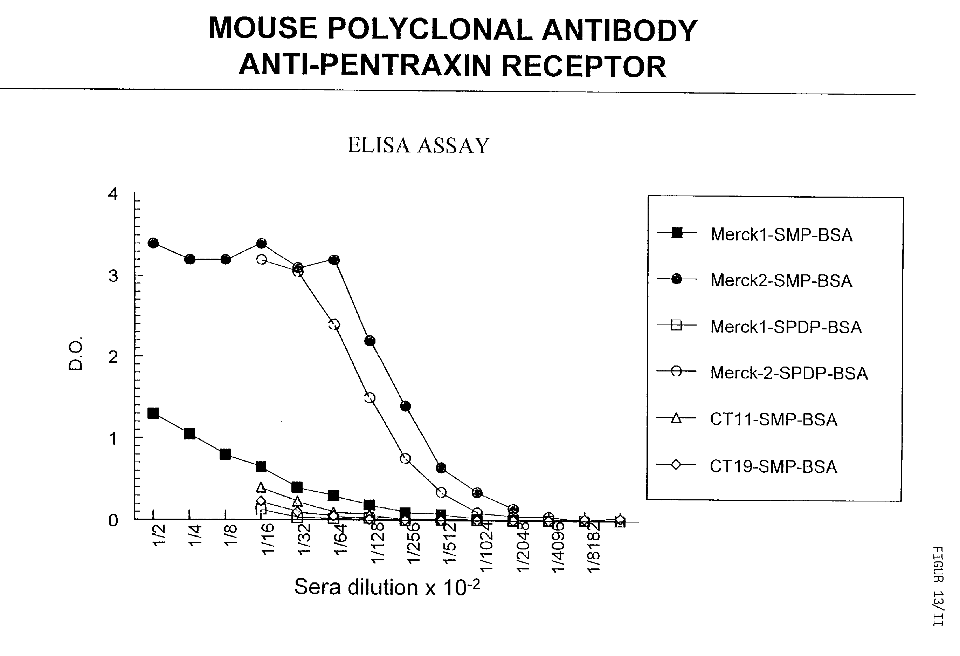

- **Figure 14:**: **Western blot with cell lines using Pentraxin I (14/I) and Pentraxin receptor (14/II) antibodies respectively**
As a negative control anti-linker and anti-peptide (Pentraxin I) or anti-peptide 2 (Pentraxin receptor) antibodies were used.

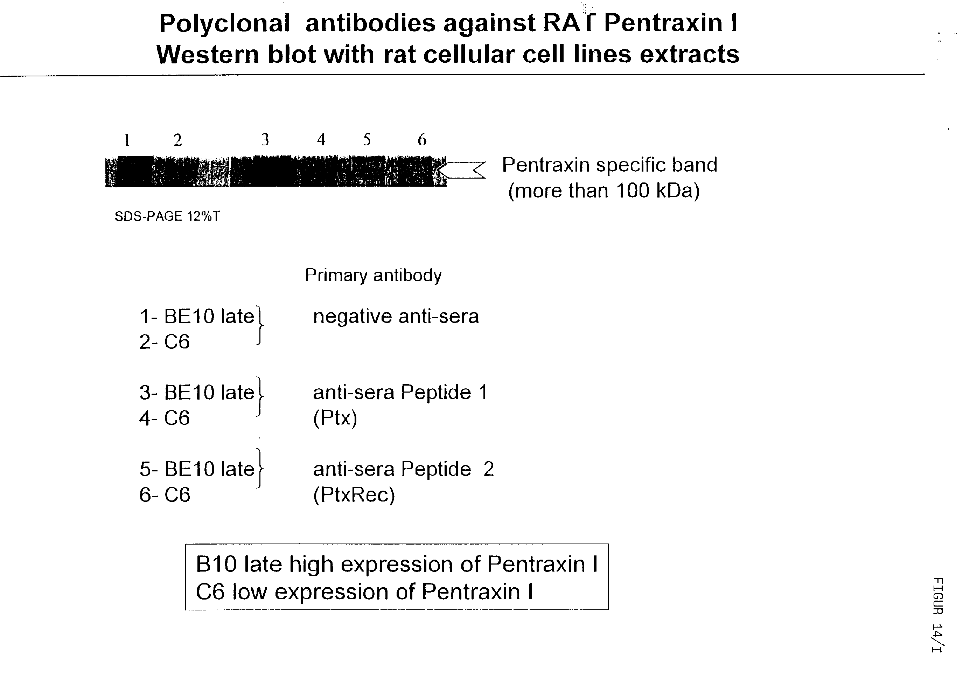

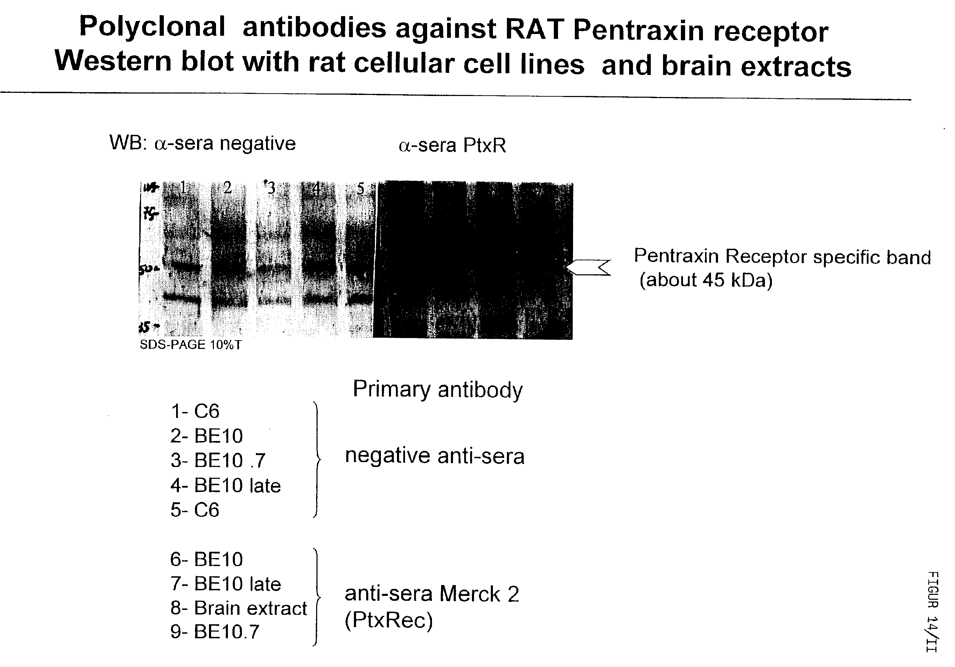

- **Figure 15:**: **Competition assay regarding rat Pentraxin I antibodies**
After western blotting of rat cell lines, the filters were incubated with anti-linker, anti-peptide 1 and anti-peptide 1 antibodies preincubated with peptide 1.
- **Figure 16:**: **Human Pentraxin receptor sequence**
The coding sequence is shown in bold. The boxed sequence corresponds to the sequence of one cDNA clone. The underligned sequence refers to synthetic oligonucleotides used for preparing the whole sequence.
- **Figure 17:**: **Human Pentraxin I sequence**
The coding sequence is shown in bold. The boxed sequence corresponds to the sequence of one cDNA clone. The underligned sequence refers to synthetic oligonucleotides used for preparing the whole sequence.

### Example 1: General procedures (In vitro models)

### (A) Cell Models: Cerebellar granule cell cultures

Primary cultures of granule cells were prepared from cerebella of 7-day-old Wistar rat pups. Dissociated cells were plated in 24 well plastic plates (2cm²) or 150 cm² flasks previously coated with poly-L-lysine hydrobromide (10 µg/ml, MW >300,000, Sigma) and the density was adjusted to give approximately 4x10⁵ cells per cm². Cells were cultured in Eagle's Basal Medium with the following additions: 10% heat-inactivated fetal calf serum (FCS), 2 mM L-glutamine, 0.1 mg/ml gentamicin, and 25 mM KCl. The replication of non-neuronal cells was prevented by adding cytosine arabinoside (10 µM) 18 to 24h after plating. The cultures were incubated at 37ºC in 5% CO₂ in air saturated with water vapor. The cells were used for the experiments described above after 8 days in culture.

### (B) Treatment of Cultures

### (I) Neuronal death evoked by potassium removal

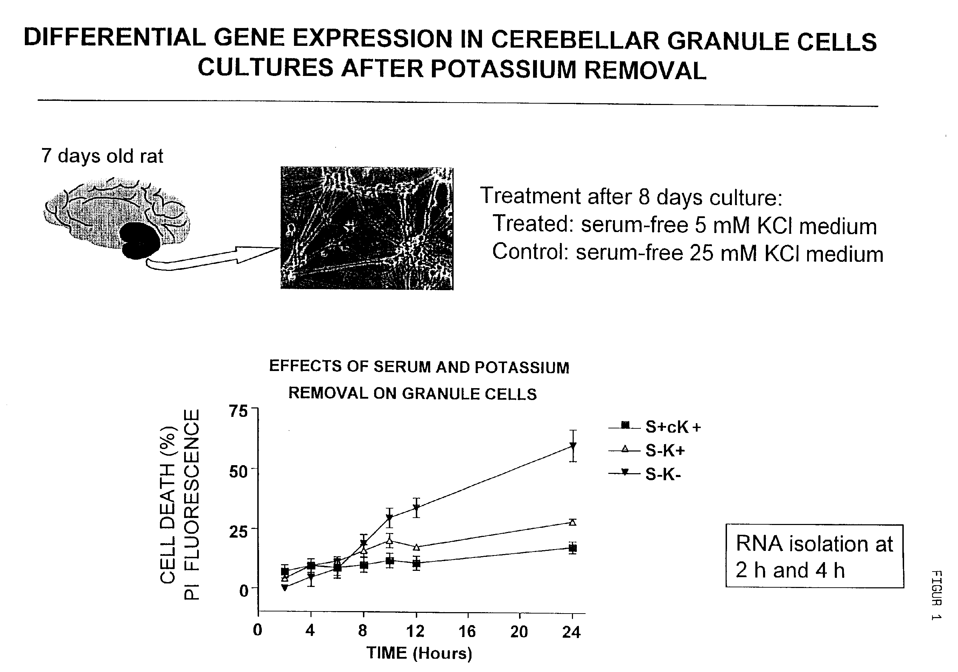

The first 8 days, the neurons were maintained in standard medium, this medium was then replaced with serum free standard medium. Neurons were washed twice and maintained in serum-free 5 mM KCl-standard medium (low potassium). Control cultures were washed identically and maintained in serum-free K25-standard medium containing 25 mM KCl. RNA was isolated according to the above description of RNA isolation from cerebellar granule cells control, 2 h and 4 h after potassium depletion (Figure 1).

### (II) Neurotoxicity induced by glutamate

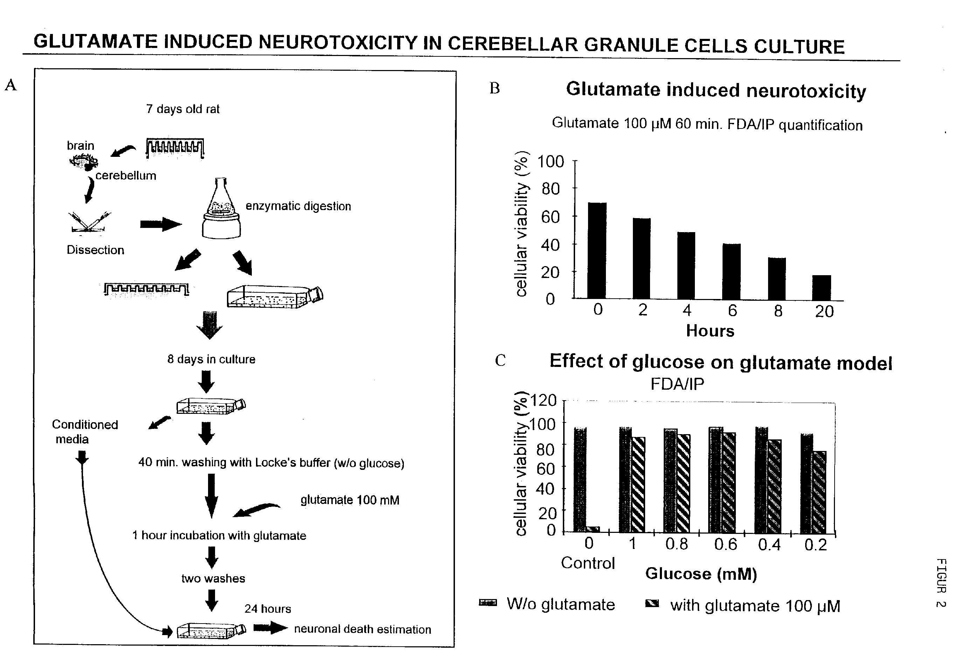

Neurotoxicity was induced in cells maintained in culture 8 days. Conditioned medium from native cultures was reserved for later use. Cultures were washed twice with modified Locke's buffer without magnesium and glucose (154 mM NaCl, 5.6 mM KCl, 2.3 mM CaCl₂ , 8.6 mM HEPES, 10 µM glycine, pH 7.4). After 40 minutes preincubation, fresh Locke's buffer containing 10 µM glycine and 100 µM glutamate was added and cultures were incubated for 1 hour at 37ºC. After then cultures were rinsed once, conditioned medium was reintroduced and cells were returned to the incubator (Figure 2). Cultures were processed for RNA extraction (see description for RNA isolation), immediately (lh sample) or after 3 hours (4h sample). For the same experiment other culture conditions were assayed: a) glucose deprivation in Locke's buffer, without glutamate induction, b) glucose, 5 mM Locke's buffer and glutamate induction. In both cases RNA extractions were done at lh and 4h. The 8 days culture was considered as a control.

### Example 2: General procedures (In vivo models)

### (A) Focal permanent ischemia

The focal permanent Stroke Model involves the occlusion of the left middle cerebral artery (MCA) in the rat following the subtemporal approach with diathermy occlusion described by Tamura et al., J. Cereb Blood Flows Metab, 1981, 53-60) and modified according to Bederson et al., Stroke 17, 1986, 472-478; Stroke 17, 1986, 1304-1307). Such approach induces a focal permanent infarction within the territory of MCA, that yields middle size infarcts (about 160 mm³ of volume) mainly restricted to cortex but with slight affection of striatum (about 5 % of whole infarct). The ipsilateral occlusion of common carotid artery contributes to improve the reproducibility of infarction. Fischer-344 rats (male, 230-260 gr.) were anaesthetized with Natrium Pentobarbital. Permanent left common carotid artery occlusion was carried out by double ligature with fine suture silk, for improving the reproducibility of infarction. Skin and temporal muscle were cut with scissors without damaging the facial nerve. The skull was opened with a drill in continuous saline flow and distal left middle cerebral artery was exposed transcranially without damage of zygomatic bone. The Dura Mater was disrupted with a 10/0 needle. The MCA was lifted off distally and occluded using fine bipolar forceps (5W). Ramifications were also occluded. Then, the artery was transacted with micro scissors to avoid recanalization. The temperature of the body was kept between 37-38ºC during the process. The brain was removed after 2, 6, 8 or 24 hours and the left and right hemispheres were frozen separately in liquid nitrogen, and the same was done for the cerebellum. Brains were stored at -80ºC until further use. Sham operated and intact animals were included as controls. The surgical process for sham rats was the same than the operated animals but the electrocoagulation of the MCA. The brain was removed after 24 hours and was treated as usual.

### (B) RNA isolation, cDNA synthesis

For RNA isolation from the stroke model, left and right hemisphere and cerebellum from intact and ischaemic rats were frozen in liquid nitrogen. Three to five samples from each treatment were pooled and RNA was extracted, according to Chomczynsky et al., Analytical Biochemistry 162, 1987, 156-159) and purified in a CsCl gradient (Sambrook et al, supra). mRNA was purified using an oligo-dT column. RNA from cell model was extracted as follows: at the indicated times the culture medium was removed from the flasks and the cells were washed twice with RNase free PBS buffer. Guanidinium isothiocyanate buffer was added directly into the flask. After gently shaking and when the cellular monolayer was disrupted, cell suspension was recovered pulling out the cells with a cell scrapper, transferred into a conical 15 ml tube and immediately disrupted through a 20 G needle. The suspension was layered over a 5.7 M CsCl solution and centrifuged at 91.000 x g for 20 h at 20º C to sediment the RNA. cDNA synthesis was carried out using the "You-prime first-Strand Beads"-kit from Amersham Pharmacia Biotech with 1 µg of mRNA and lmM of the appropriate oligonucleotides. All the steps involving RNA manipulation were done with DEPC treated material and solutions according to general protocols (Sambrook et al., supra)

### (C) Differential Display

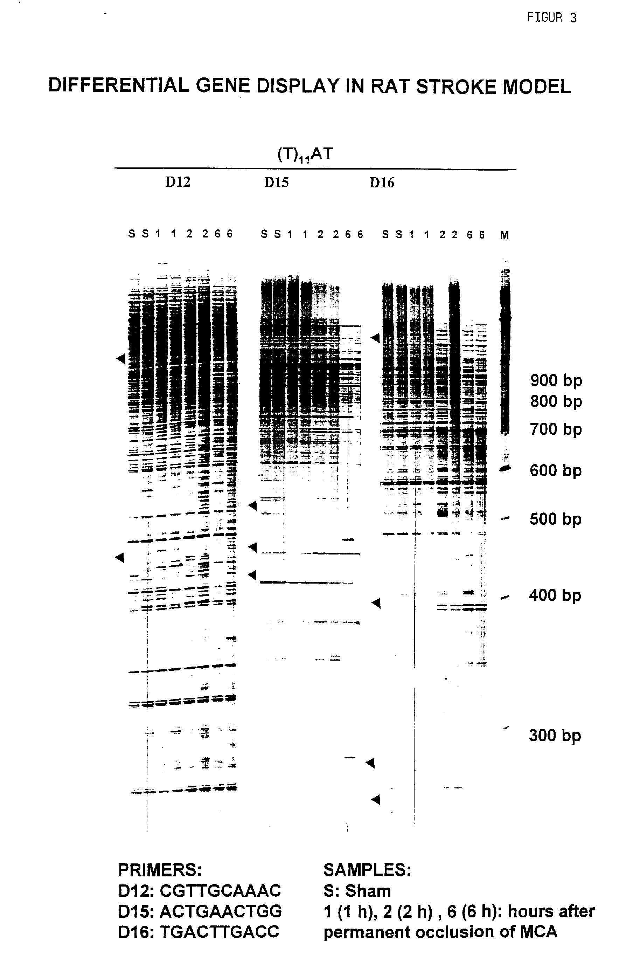

RNA from the stroke model (sham, 2 and 6 hours from left hemisphere) and the granule cells under serum and potassium deprivation (control, 2 and 4 hours) were used in the "Differential Display"(DD)-experiments. The method used was the originally described (Liang and Pardee, Science 257, 1992, 967-971). The 12 oligonucleotides used for sscDNA synthesis were (dT)₁₁VN (downstream oligonucleotides). Amplification of the sscDNA was conducted with the same 3' oligonucleotide and a second decamer arbitrary 5' oligonucleotide (upstream oligonucleotide). PCR amplification products were analyzed on a denaturing 6% polyacrylamide gel (Genomyx) in a Genomyx LR apparatus. Gels were run at 800V for 16 h. DNA was visualized by silver staining, and dried (Figure 3). DNA bands corresponding to differentially regulated genes were cut out from the gel. DNA was eluted from the gel by immersion in 10 ml of H₂O for 2 h at -20C, and reamplified using the same pair of oligonucleotides and the same PCR conditions. The resulting bands were directly cloned into the "pGem-T Easy"-vector (Promega), and three positive clones sequenced (Silver sequence system from Promega). In the case that the three clones had the same sequence, it was assumed that they correspond to the band selected from the gel and there was only one. Sequences were compared to genomic databases. Validation of the obtained bands was done initially by Reverse Northern. Positive ones were further analyzed by Northern and/or semi-quantitative PCR.

### (D) Reverse Northern and Northern blots

Selected bands from the DD were electrophoresed in a 1.5% agarose gel and transferred to a nylon membrane (Sambrook et al, supra). The membranes were hybridized with the appropiate dscDNA labeled with ³²P for each model used in the DD experiments, using actin as a control of the signal. Bands from each hybridization experiment were referred to actin and compared to the corresponding one. Northern blots were performed with 5 µg of mRNA samples from the stroke model (left and right hemisphere and cerebellum from intact and sham, and 2 h, 6 h, 8 h, 24 h, 7d, 14 d and 21 d ischaemic rats), the cell cultures under glutamate treatment or the cells deprivated of serum and potassium. Rat Pentraxin I and Pentraxin receptor, respectively, was radioactively labeled and used as a probe. mRNA amount per sample was checked using actin. Membranes from human tissues and dissected brains were purchased from Clontech. Hybridizations were done with actin and a subclone of human Pentraxin I.

### (E) Cloning procedures

### Pentraxin I:

Human Pentraxin I was cloned by PCR using a set of specific oligonucleotides corresponding to the coding region. The complete cDNA was previously published (mouse and human neuronal Pentraxin I (Perin et al, supra)) and the sequence can be found in the GenBank database (accession U61849). This allowed to design the specific primers for PCR cloning. Human RNA samples from brain cortex, hippocampus and cerebellum as well as cDNA from total brain purchased from Clontech were used.

The following primers were used:

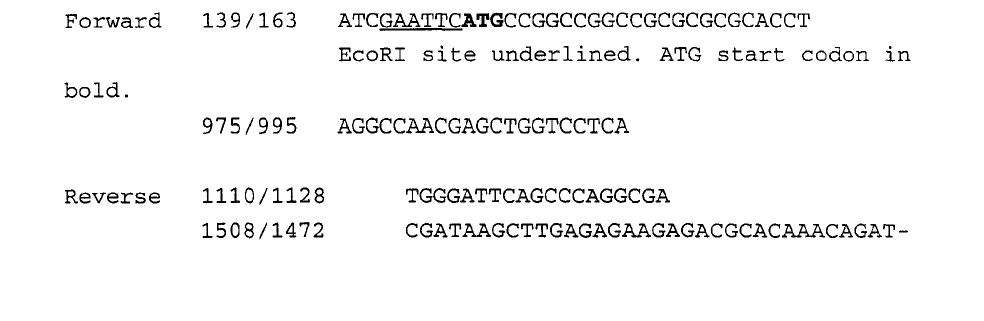

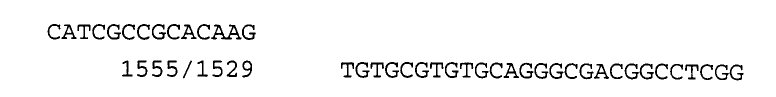

Due to the length of the mRNA ( about 5.5 kb), the primers were designed only for the coding region (bases 138 to 1431). Several cDNA clones were obtained. One of these clones includes a fragment from base 723 to 1328 (Figure 17).

### Human pentraxin receptor:

For full length cloning, it was necessary to obtain a cDNA sequence of the human Pentraxin receptor. The cDNA sequence for the human Pentraxin receptor was deduced from a genomic sequence found in the EMBL databank (accession number: HS327J16) using the sequence from the rat Pentraxin receptor previously published (Dodds et al., J. Biol. Chem. 272, 1997, 21488-21494) (accession number: AF005099). A homology search was conducted in public data banks and fragments of one genomic sequence were found that showed homology to the coding region of the gene (accession AL008583). The fragments corresponded to putative exons (Figure 24). They were all put together and the final result was a sequence with 85% similarity in the coding region with the rat sequence (Figure 25). This allowed to design specific primers for PCR cloning. Human brain tissue and brain cDNA purchased from Clontech were used.

The following primers were used:

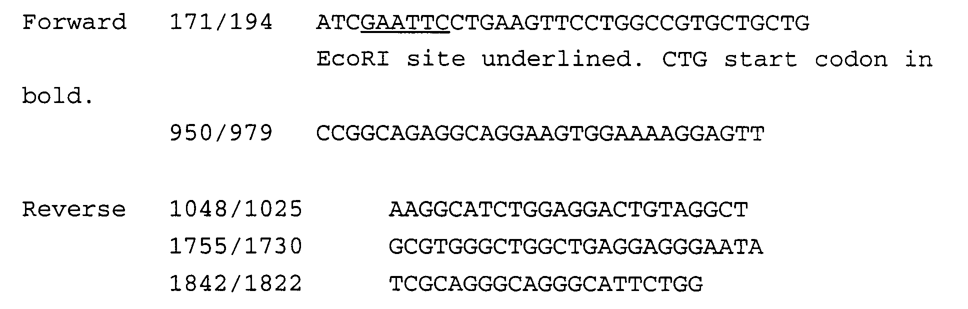

Several cDNA clones were obtained. One of these clones includes a fragment from base 950 to 1755 (Figure 16).

### (F) Semi-quantitative PCR

sscDNA was synthesized using a mix of (dT)₁₂A, (dT)₁₂C and (dT)₁₂G 1 µM each of mRNA samples from the stroke model (left and right hemisphere from intact, sham, and 2h, 8h and 24h ischaemic rats) and from granule cells under glutamate treatment. For PCR amplification, rat Pentraxin I (forward 1390/1410: AGGCCAACGAGCTGGTCCTCA; reverse 1795/1777: TGGGATTCAGCCCAGGCGA), rat Pentraxin receptor (forward: 950/979: CCGGCAGAGGCAGGAAGTGGAAAAGGAGTT; reverse 1574/1549: GCCTCTACCAGCTTGTCTTCCCAGGG) and actin (forward: CGATATCGCTGCGCTCGTCGT; and reverse: ATCTCCTTCTGCATCCTGTC) -as internal control- were used. Forward primers were labelled with biotin-UTP. Samples were removed from each cycle from the 15th to the 30th in order to determine the exponential phase of the gene amplification. The PCR product was electrophoresed and transferred to a nylon membrane. After quimioluminiscent detection, the films were scanned, and the intensity of the bands was measured. Ratios between actin and Pentraxin I or Pentraxin receptor were computed, and the values of the same cycle for different samples were compared.

### (G) In situ hybridization

Rats subjected to MCAO were sacrificed at 6 and 24 hours, and 7 days post occlusion and perfused with 0.1 M phosphate buffer followed by 4% paraformaldehyde in 0.1 M phosphate buffer. Brains were dissected out, post-fixed in the same fixative for 8 hr at 4ºC and cryoprotected in 10% sucrose solution. 20 µm thick coronal sections were cut in a cryostat, mounted in sylane treated slides and stored at -40ºC. Intact rats were used as control.

Plasmid DNA containing a rat Pentraxin I fragment or a rat Pentraxin receptor fragment was linearized with selected restriction enzymes, phenol/chloroform extracted and ethanol precipitated. The pellet was resuspended in 25 µl of H₂O, and the concentration measured under UV. 1 µg of purified linearized plasmid was transcribed using either T7, T3 or Sp6 RNA polymerases with the "Dig RNA labeling"-kit from Roche Diagnostics. The yield of labeled RNA was estimated using labeled control RNA from the same kit in a dot blot procedure. Riboprobes were used at 250 to 500 ng/ml.

Before hybridization, sections were sequentially hydrated in PBS and incubated with 0.2% of Triton-X100 in PBS. Sections were then treated with 0.2 N HCl (10 min) and acetylated in 0.1 M triethanolamine, 0.25% acetic anhydride (5 min). Then, sections were post-fixed in 4% paraformaldehyde (10 min), washed in PBS, rinsed in 0.025 M glycine (5 min) and sequentially dehydrated in ethanol (50,70,90 and 100%). Sections were prehybridized with hybridization buffer containing 50% formamide, 20% dextran sulfate, 5% Denhardt solution, 2% 0.5 M EDTA, 2% 1 M PIPES, 0.2% SDS, 5% 1 M DTT, 2.5% 10 mg/ml ssDNA, 2.5% 10 mg/ml yeast-tRNA and 10% 20xSSC (2hr at 60ºC) and subsequently hybridized in the same buffer containing 400 ng/ml probe (overnight at 60ºC). After hybridization, sections were washed twice in 2xSSC and treated with 20 µg/ml RNase A in 0.01 M Tris-HCl pH 7.5 (1 hr, 37ºC). After, sequential stringency washes were done with 2xSSC, lxSSC, 0.5xSSC (15 min each at room temperature) and 0.1xSSC (30 min at 60ºC).

Sections were subsequently blocked with 1% BSA in Tris-buffer pH7.5 (2 h at room temperature) and incubated with sheep polyclonal anti-digoxigenin antiserum conjugated with alkaline phosphatase in Tris-buffer pH7.5 (overnight at 4ºC). Then, sections were washed and color was developed incubating with BCIP/NBT for several hours.

### (H) Design of synthetic peptides and conjugation

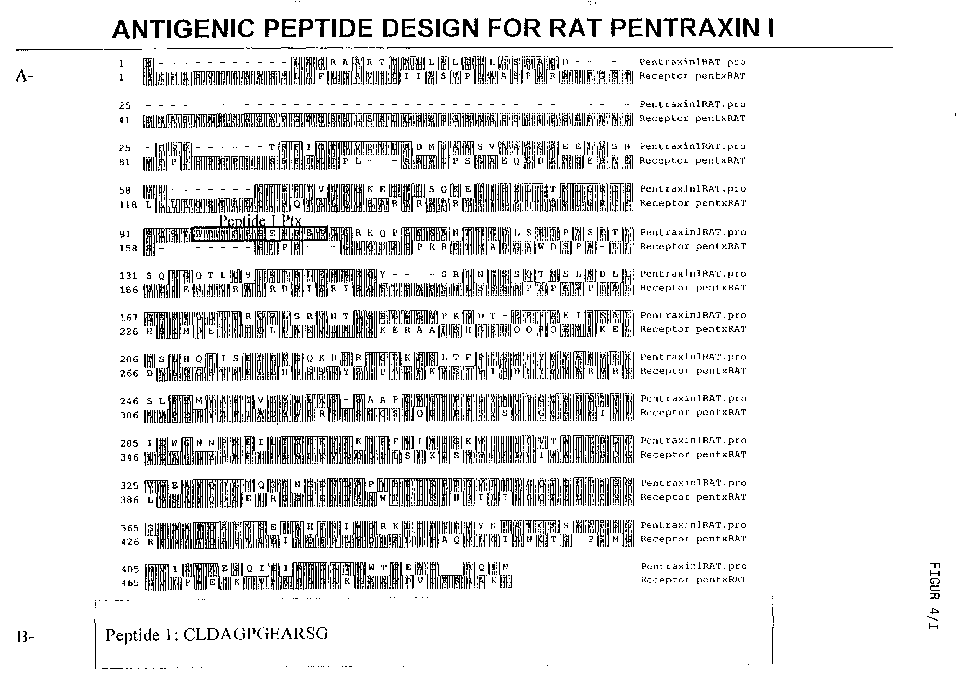

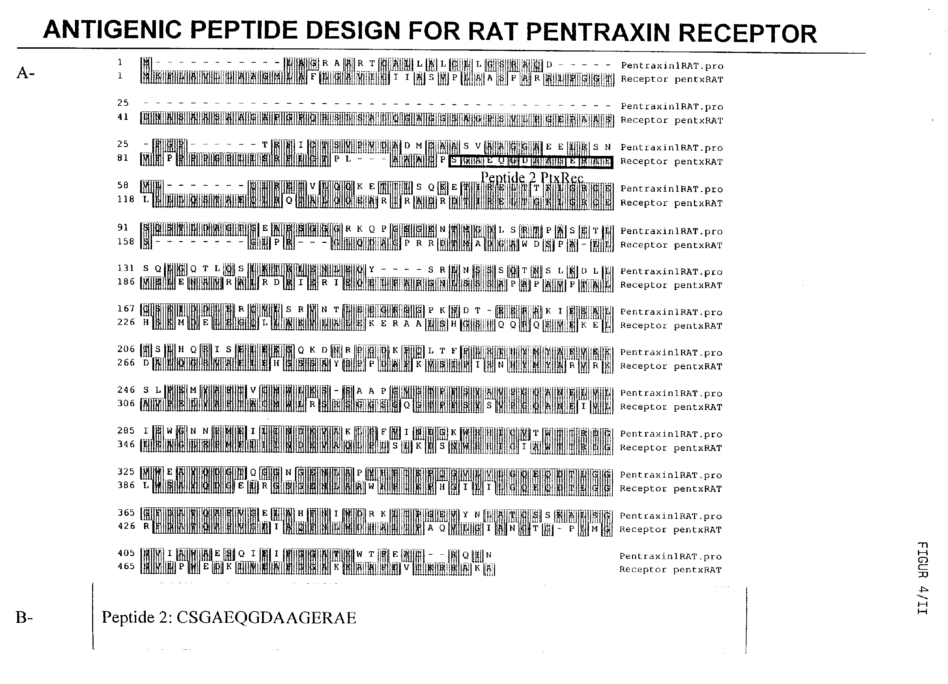

A synthetic peptide was designed for the raising of specific antibodies based on the sequence of Rat Pentraxin I and the rat Pentraxin receptor, respectively. For the design two principal aspects were considered: the high homology between the members of the pentraxin family and the accessibility of the aminoacids residues. Figure 4 shows the sequence of the peptides. For the prediction of protein structures conventional methods were used.

Synthetic peptides were linked to a carrier (BSA or KLH) using two commercial linkers: SMP (N-succinimidyl 3-maleimidopropionate) and SPDP (N-succinimidyl 3-(2-pyridyldithio-propionate) both from Pierce following the manufacturer recommendations.

### (I) Production of antibodies

Polyclonal antibodies against synthetic peptides were obtained after intraperitoneal (i.p.) administration of conjugated KLH peptides and Ribi as adjuvant in balb/c mouse. A second dose was administrated 21 days after the first one, and the serum was extracted at day 31. 50µg of peptide was used per dose and 10 animals per peptide. The presence of specific antibodies in the serum was tested using a standard ELISA method. In order to avoid a cross-reaction the coating of ELISA plates was performed using SMS-BSA peptide and peptide-SPDP-BSA at 1 µg/ml. In case of monoclonal antibodies, B cells from the spleen of immunised mouse were fused with Friendly Myeloma-653 (Vertex) cells as a partner using PEG following the standard method (Kohler and Milstein, supra).

### (J) SDS-PAGE and Immunoblotting

Sodium dodecyl sulfate polyacrylamide gel electrophoresis (SDS-PAGE) was performed according to Laemmli's method. Proteins were transferred onto nitrocellulose membranes. The presence of Pentraxin I and Pentraxin receptor in cells or tissue extracts were determined by Western Blot after incubation of polyclonal serum (diluted 1/2000) or hybridoma supernatants followed by incubation of a conjugated antibody.

### (K) Antisense assay

The effects of sense and antisense oligonucleotides against rat Pentraxin I were investigated on low potassium induced cell death in cerebellar granule neurons. The phosphorothioate analoges of 21-mer antisense and sense oligodeoxyribonucleotides to the rat Pentraxin I transcript used were as follows:

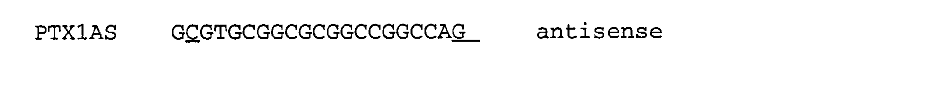

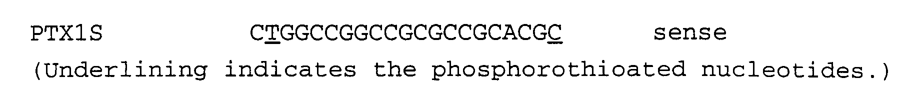

A concentration of 10 µM ODN was found to be optimal for "antisense knockdown". Either sense or antisense ODN were added immediately after switching granule cell cultures from high to low potassium media. Viable neurons were quantified by simultaneously staining with two fluorescent dyes, fluorescein diacetate (FD) and propidium iodide (PI), 24 h after potassium deprivation. The stained cells were examined with a standard epi-illumination fluorescence microscope and pictures were taken. Neuronal cell death was expressed as % cell death obtained as follows: PI positive cells X 100 / (PI positive + FD positive cells).

### Example 3: Expression of the Pentraxin I gene in a stroke model and an in vitro apoptotic model

The stroke model was validated for later DD studies by hybridization of samples from both hemispheres -ipsilateral and contralateral- after MCAO with β-actin as a control, and the heat shock protein 70 (HSP70), described as an immediate-early gene induced after MCAO (Figure 5). There was a weak signal in the left hemisphere at 30 min after the MCAO, reaching its maximum at 2 hours and lasting at least for 8 hours. No expression was seen in the contralateral hemisphere. According to these results, samples from sham, 2 and 6 hours after MCAO were selected for the DD experiments.

An *in vitro* apoptotic model was set up with the same objective. Switching culture conditions of cerebellar granule cells at 8 days *in vitro* from medium containing high KC1 (25 mM) to medium containing low concentrations (5 mM) resulted in time dependent increase in neuronal cell death measured by propidium iodide fluorescence (Figure 1). The loss of neurons 24 h after lowering the potassium concentration and serum removal was approximately 60%, compared to 15% death in controls and 20% death after serum removal in the presence of 25 mM potassium. No significant differences in cell death were observed among these groups 2 h and 4 h after treatment. Total RNA of each treatment group at these time periods was used to investigate differential gene expression.

In both experimental models, a selected band from DD experiments showed high homology to the previously described human and rat neuronal Pentraxin I. The band from the stroke model was homologous to the rat Pentraxin I from base 1479 to 1877 (stop at base 1853), and the one from the apoptotic model from base 5127 to 5339. However, in the rat stroke model this band was selected because there was a decreasing signal during the time course, but in the *in vitro* apoptotic model there was an increase of intensity. Northern analysis confirms this result in the apoptotic model (Figure 6) showing an upregulation in cultures depleted of serum and KCl, but in the stroke model it was not possible to see clear differences in the expression of Pentraxin I (Figure 7/I), even in cases when sequences from the non-codifying regions were used as a probes.

### Example 4: Expression of the Pentraxin receptor gene in a stroke model

The stroke model was validated for later DD studies by hybridization of samples from both hemispheres -ipsilateral and contralateral- after MCAO with β-actin as a control, and the heat shock protein 70 (HSP70), described as an immediate-early gene induced after MCAO (Figure 5). There was a weak signal in the left hemisphere at 30 min after the MCAO, reaching its maximum at 2 hours and lasting at least for 8 hours. No expression was seen in the contralateral hemisphere. According to these results, samples from sham, 2 and 6 hours after MCAO were selected for the DD experiments. A selected band from the DD experiments showed high homology to the previously described rat reuronal Pentraxin receptor. The band showed homology to the rat Pentraxin receptor from base 2495 to 3057 (stop at base 1631). This band was selected because there was an increase of intensity. By Northern analysis it was not possible to see clear differences in the expression of Pentraxin receptor (Figure 7/II).

### Example 5: Tissue distribution of Pentraxin I mRNA

Hybridization of Northern dot blots with a fragment corresponding to bases 723 to 1048 (coding region) of the human Pentraxin I gene showed that Pentraxin I was distributed mainly in the whole brain, and weak signals were apparent in pituitary and adrenal glands. In the brain, the expression was seen in the cerebral cortex, amygdala, hippocampus, occipital lobe and cerebellum, and in a lesser extent, in the putamen (Figure 8/I). Also, the expression was studied in some human and rat glyoma and neuroblastoma cell lines. High expression was detected by PCR amplification in the human cell line T98 G (Figure 9/I), and only a weak band in the rat neuroblastoma cell lines BE 10 and BE 10.7 (Figure 10/I). *In situ* hybridisation using riboprobes generated from a fragment from base 1390 to 1795 of the rat Pentraxin I gene showed the same distribution of the signal as the Northern dot blots (Table 1). Also, it was possible to see an increase of the signal both in the ipsilateral and contralateral hemispheres in the cortex and hippocampus. In the cerebellum, this increase was less apparent.

### Example 6: Tissue distribution of Pentraxin receptor mRNA

Hybridization of Northern dot blots with a fragment corresponding to bases 1029 to 1755 showed that Pentraxin receptor was distributed mainly in the whole brain. The expression was seen in the cerebral cortex, amygdala, hippocampus, occipital, frontal and temporal lobes and cerebellum, and in a lesser extent, in the putamen (Figures 8/II, III). Also, the expression was studied in some human and rat glyoma and neuroblastoma cell lines. High expression was detected by PCR amplification in the human cell lines U 186 MG and U 87 MG (Figure 9/II), and in the rat neuroblastoma cell lines BE 10, BE 10.7 and BE 10.7 late and in the glyoma C6 (Figure 10/II). *In situ* hybridisation using riboprobes generated from a fragment from base 2495 to 3057 of the rat Pentraxin receptor gene showed the same distribution of the signal as the Northern dot blots (Table 2). Also, it was possible to see an increase of the signal both in the ipsilateral and contralateral hemispheres in the cortex and hippocampus. In the cerebellum, this increase was less apparent.

### Beispiel 7: Regulatory mechanisms of expression of the Pentraxin I gene

In order to study the possible regulatory mechanisms of Pentraxin I expression, the effect on the transcript level of glucose and/or glutamate in cerebellar granule cells was studied. As it is shown in Figure 2, panel B, the excitotoxic effect of glutamate, measured as cell death, is seen when there is glucose deprivation prior and during glutamate treatment. Semi-quantitative PCR studies showed (Figure 11/I) that in cells deprived of glucose (lanes 2 and 3) there is a decrease of Pentraxin I mRNA levels during the first 4 hours. In cells deprived of glucose and treated with glutamate (lines 4 and 5) there was an increase of the mRNA levels up to control in both culture times. This effect was also seen in cases in wich the glutamate treatment was done in presence of glucose (lanes 6 and 7), but later than the former. Then, it can be concluded that glutamate induces the expression of Pentraxin I irrespective on the effects on cell viability, and glucose deprivation represses its expression.

### Beispiel 8: Regulatory mechanisms of expression of the Pentraxin receptor gene

In order to study the possible regulatory mechanisms of Pentraxin receptor expression, the effect on the transcript level of glucose and/or glutamate in cerebellar granule cells was also studied. Semi-quantitative PCR studies showed (Figure 11/II) that in cells deprived of glucose (lanes 2 and 3) there is a fast decrease of Pentraxin receptor mRNA levels during the first 2 hours. At 4 hours there is an increase, but the levels are still below the control levels. In cells deprived of glucose and treated with glutamate (lines 4 and 5) there was an slight increase of the mRNA levels up to control in both culture times. However, in cases in which the glutamate treatment was done in presence of glucose (lanes 6 and 7), there was an increase up to control level at 4 hours. Then, it can be concluded that glutamate also induces the expression of Pentraxin receptor irrespective on the effects on cell viability, and glucose deprivation represses its expression.

### Example 9: Pentraxin I expression in the presence of antisense RNA

An oligonucleotide (sense and antisense) corresponding to bases 561 to 581 of the Pentraxin I gene was used to see the effect on the cell survival in the apoptotic model. As it is shown in Figure 12, only antisense treatment induces a significant reduction of the percentage of cell death. This is indicative that repression of the expression of the Pentraxin I encoding gene has a neuroprotective effect.

### Example 10: Generation of specific anti-Pentraxin I antibodies

Polyclonal antibodies were obtained using a synthetic peptide homologous to rat Pentraxin I sequence. A specific peptide, peptide 1 (Figure 4/I, panel B), was conjugated to KLH for the immunization and to BSA for the screening (Figure 13/I). The presence of specific antibodies was detected using for the coating the peptide 1 conjugate to BSA by means of two different linkers, SMP and SPDP. A specific band around 100 kDa was detected after western blotting with extracts of two neuronal rat cell lines (Figure 14/I). The 100 kDa band obtained in the western blot after the incubation with the anti-peptide 1 anti-sera disappears if this anti-sera was preincubated with peptide 1 (Figure 15).

### Example 11: Generation of specific anti-Pentraxin receptor antibodies

Polyclonal antibodies were obtained using a synthetic peptide homologous to rat Pentraxin receptor sequence. A specific peptide, peptide 1 (Figure 4/II, panel B), was conjugated to KLH for the immunization and to BSA for the screening (Figure 13/II). The presence of specific antibodies was detected using for the coating the peptide 1 conjugate to BSA by means of two different linkers, SMP and SPDP. A specific band around 45 kDa was detected after western blotting with extracts of all neuronal rat cell lines (Figure 14/II).

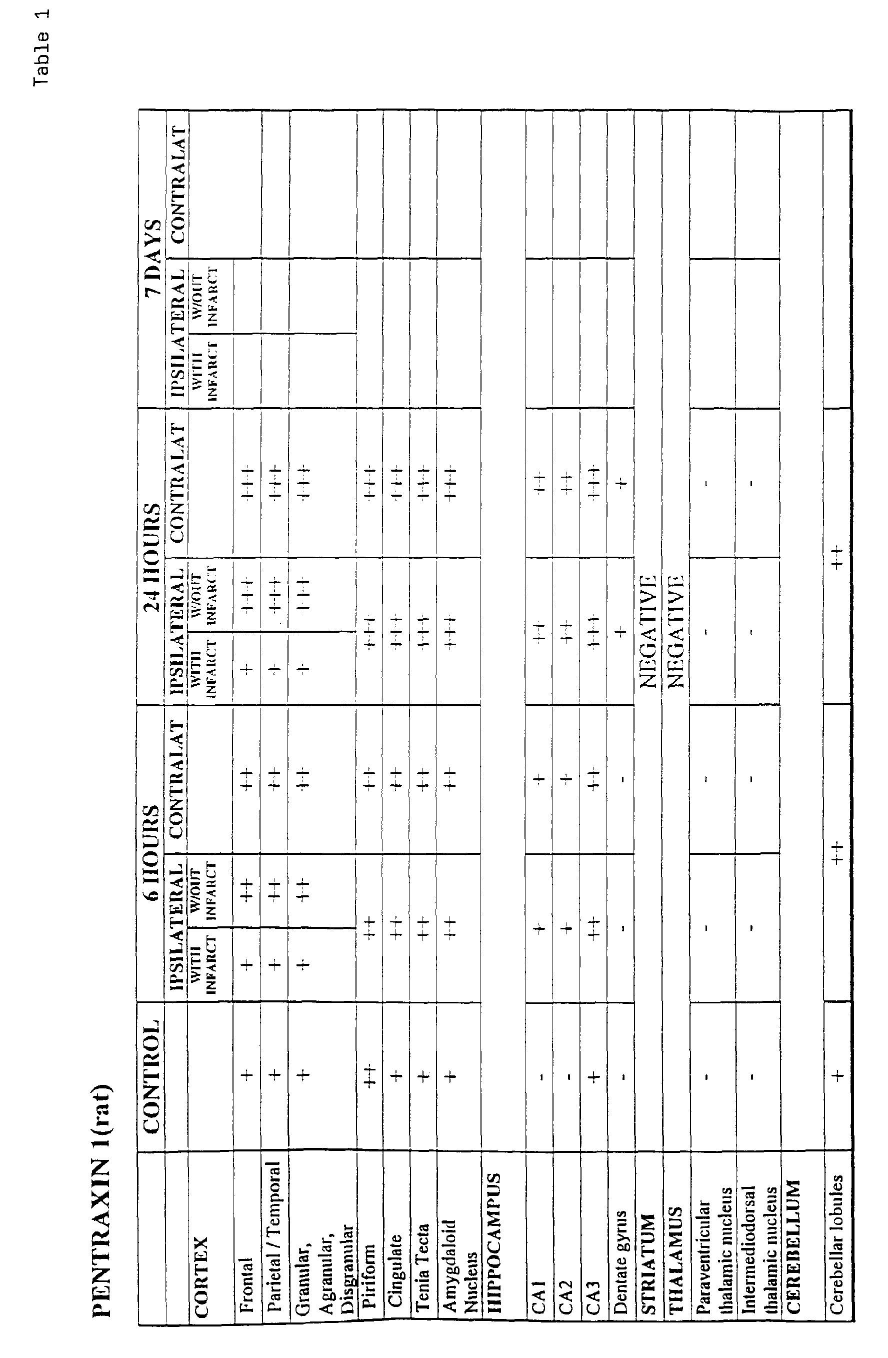

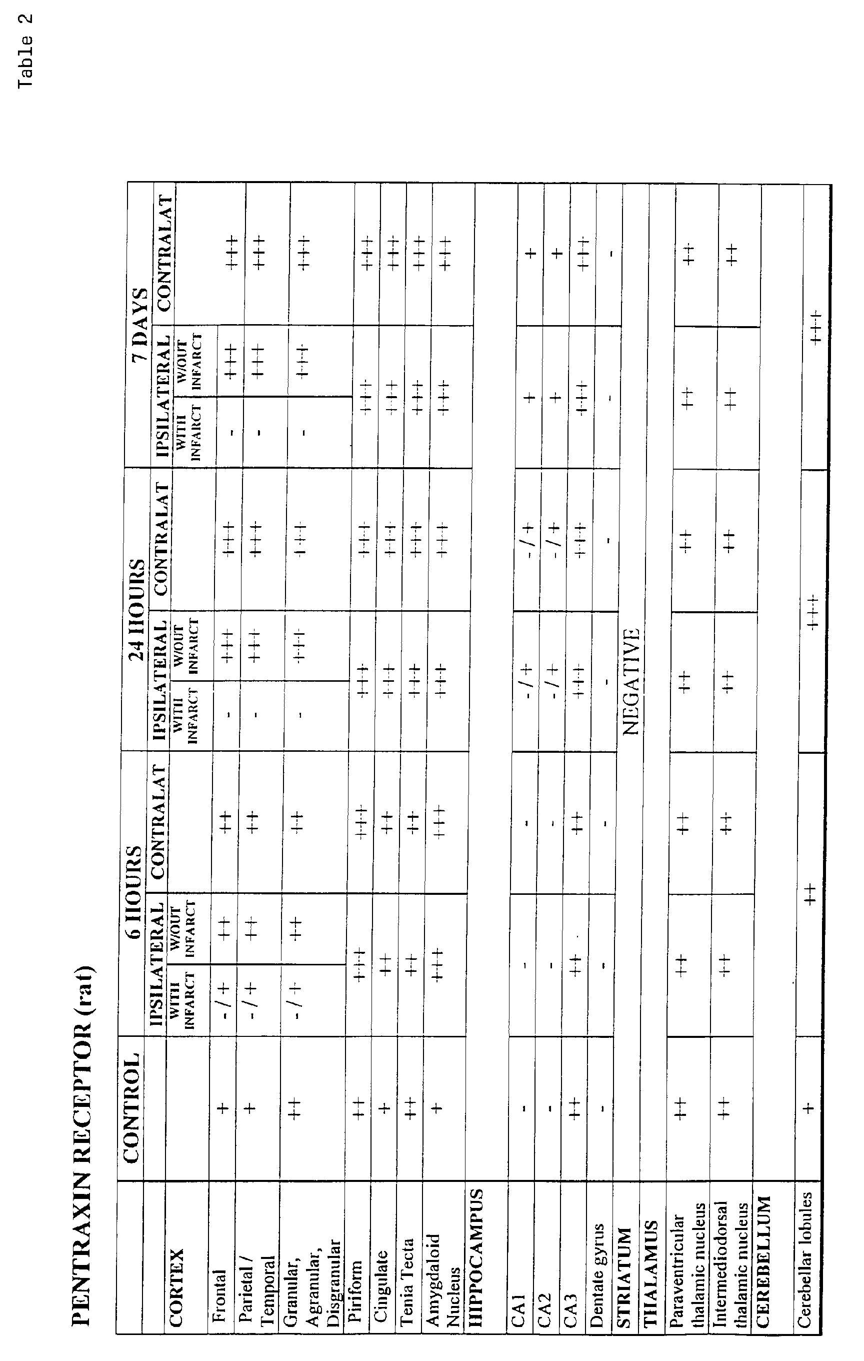

## Claims

1. A nucleic acid sequence encoding the human Pentraxin receptor or a protein exhibiting biological properties of the human Pentraxin receptor and being selected from the group consisting of
(a) a nucleic acid sequence comprising the coding region of the nucleotide sequence depicted in Figure 16;
(b) a nucleic acid sequence which hybridizes to a nucleic acid sequence specified in (a);
(c) a nucleic acid sequence which deviates from the nucleic acid sequences specified in (a) and (b) due to the degeneracy of the genetic code; and
(d) a nucleic acid sequence, which represents a fragment, derivative or allelic variation of a nucleic acid sequence specified in (a) to (c).

2. A recombinant vector containing the nucleic acid sequence of claim 1.

3. The recombinant vector of claim 2 wherein the nucleic acid sequence is operatively linked to regulatory elements allowing transcription and synthesis of a translatable RNA in prokaryotic and/or eukaryotic host cells.

4. A recombinant host cell containing the recombinant vector of claim 3.

5. The recombinant host cell of claim 4, which is a mammalian cell, a bacterial cell, an insect cell or a yeast cell.

6. A protein exhibiting biological properties of the human Pentraxin receptor which is encoded by a nucleic acid molecule of claim 1.

7. A method of producing a protein exhibiting biological properties of the human Pentraxin receptor comprising:
(a) culturing the recombinant host cell of claim 4 or 5 under conditions such that said protein is expressed; and
(b) recovering the protein.

8. A protein produced by the method of claim 7.

9. An antibody directed to the protein of claims 6 or 8.

10. A pharmaceutical composition comprising a therapeutically effective amount of at least one of the following compounds:
(a) Pentraxin I or the protein encoded by the nucleic acid sequence of claim 1,
(b) a nonfunctional variant of the protein of (a); (c) an antibody to the protein of (a) or (b);
(d) a nucleic acid sequence encoding the protein of (a) or (b);
(e) an antisense RNA sequence characterized in that it is complementary to an mRNA transcribed from a gene encoding Pentraxin I, the nucleic acid sequence of claim 1 or a part thereof and can selectively bind to said mRNA or part thereof, said sequence being capable of inhibiting the synthesis of Pentraxin I or human Pentraxin receptor or a protein having the biological properties of the human Pentraxin receptor; or
(f) a ribozyme characterized in that it is complementary to an mRNA transcribed from a gene encoding Pentraxin I, the nucleic acid sequence of claim 1 or a part thereof and can selectively bind to and cleave said mRNA or part thereof, thus inhibiting the synthesis of Pentraxin I or the human Pentraxin receptor or a protein having the biological properties of the human Pentraxin receptor;
optionally in combination with a pharmaceutically acceptable carrier.

11. Use of one of the compounds as defined in claim 10 for the preparation of a pharmaceutical composition for preventing, treating or ameliorating a disease associated with a neuronal disorder or for providing a neuroprotective effect.

12. Use of claim 11, wherein the neuronal disorder is a stroke, an acute head trauma, multiple sclerosis, a spinal cord injury, Alzheimer disease or a brain tumor.

13. A method for detecting a disease associated with a neuronal disorder based on a deregulated expression of Pentraxin I or human Pentraxin receptor or the presence of Pentraxin I or human Pentraxin receptor having no or at least reduced biological activity comprising contacting a target sample suspected to contain Pentraxin I, human Pentraxin receptor or Pentraxin I or human Pentraxin receptor encoding nucleic acid sequences with a reagent which reacts with Pentraxin I or human Pentraxin receptor or Pentraxin I or the human Pentraxin I encoding nucleic acid sequences and detecting abnormal amounts or abnormal forms of Pentraxin I or the human Pentraxin receptor or Pentraxin I or human Pentraxin receptor encoding nucleic acid sequences.

14. The method of claim 13, wherein the reagent is a polynucleotide capable of specifically hybridizing to Pentraxin I or human Pentraxin receptor encoding nucleic acid sequences.

15. The method of claim 14, wherein the reagent is an anti-Pentraxin I-antibody or an anti-human Pentraxin receptor-antibody.

16. The method of any one of claims 13 to 15, wherein the reagent is detectably labeled.

17. A diagnostic kit comprising (a) probe(s) for detecting abnormal amounts or abnormal forms of Pentraxin I and/or human Pentraxin receptor or Pentraxin I and/or human Pentraxin receptor encoding nucleic acid sequences.

18. The kit of claim 17, wherein the probe is an anti-Pentraxin I-antibody or an anti-human Pentraxin receptor-antibody or a polynucleotide capable of specifically hybridizing to the Pentraxin I or the human Pentraxin receptor encoding nucleic acid sequences.
